# EUROPÄISCHE PATENTSCHRIFT

(11) **EP 2 296 635 B1**
(45) Veröffentlichungstag und Bekanntmachung des Hinweises auf die Patenterteilung: **06.09.2017**
(21) Anmeldenummer: 09777024.2
(22) Anmeldetag: 08.07.2009
(51) Int. Cl.: A61K 9/50, A61K 9/51, A61K 9/16, A61K 31/755, A01N 1/02, A61K 9/00

(54) **KÜNSTLICHE SAUERSTOFFTRÄGER UND IHRE VERWENDUNG**
ARTIFICIAL OXYGEN CARRIERS AND USE THEREOF
TRANSPORTEURS D'OXYGÈNE ARTIFICIELS ET LEUR UTILISATION

(30) Priorität: 09.07.2008 DE 102008032183; 30.08.2008 DE 102008045152
(43) Veröffentlichungstag der Anmeldung: 23.03.2011
(73) Patentinhaber: Universität Duisburg-Essen, 45141 Essen (DE)
(72) Erfinder: DE GROOT, Herbert, 40597 Düsseldorf (DE); MAYER, Christian, 47269 Duisburg (DE); PETRAT, Frank, 45277 Essen (DE); KIRSCH, Michael, 40723 Hilden (DE)
(74) Vertreter: Von Rohr Patentanwälte Partnerschaft mbB
(86) Internationale Anmeldenummer: PCT/EP2009/004925
(87) Internationale Veröffentlichungsnummer: WO 2010/003647

(56) Entgegenhaltungen:
- WO-A1-94/18954
- WO-A1-2007/134304
- US-A- 5 490 986
- US-A- 5 662 932
- US-A1- 2006 182 807
- PISANI E; TSAPIS N; PARIS J; NICOLAS V; CATTEL L; FATTAL E: "Polymeric nano/microcapsules of liquid perfluorocarbons for ultrasonic imaging: physical characterization" LANGMUIR : THE ACS JOURNAL OF SURFACES AND COLLOIDS, Bd. 22, Nr. 9, 25. April 2006 (2006-04-25) , Seiten 4397-4402, XP002582007 United States ISSN: 0743-7463 in der Anmeldung erwähnt

## Beschreibung

Die vorliegende Erfindung betrifft das Gebiet der künstlichen Sauerstoffträger, insbesondere als Blutersatzstoffe für den menschlichen oder tierischen Körper. Insbesondere betrifft die vorliegende Erfindung künstliche Sauerstoffträger auf der Basis von perfluorcarbonhaltigen Kapseln, insbesondere Nanokapseln.

Insbesondere betrifft die vorliegende Erfindung künstliche Sauerstoffträger in Form von Dispersionen, welche sich insbesondere als Blutersatzstoffe für den menschlichen oder tierischen Körper, vorzugsweise zu Zwecken der Transfusion, eignen.

Des weiteren betrifft die vorliegende Erfindung die Verwendung dieser Dispersionen insbesondere als Blutersatzstoffe für den menschlichen oder tierischen Körper, insbesondere zu Transfusionszwecken, beispielsweise zur Behandlung von Zuständen nach Blutverlusten, ischämischen Zuständen und Zuständen nach Reperfusion, oder aber zu anderen Anwendungszwecken.

Blut, das für Transfusionen zur Verfügung steht, ist ein knappes Gut. Bluttransfusionen sind bei Unfallopfern, bei chirurgischen Eingriffen und bei der Therapie chronischer Anämien häufig lebensrettende Maßnahmen. Die Verfügbarkeit von Blutkonserven wird unter anderem aufgrund von Infektionsrisiken (z. B. HIV, Prionen, Hepatitis A, B oder C etc.) und nachlassender Spenderbereitschaft immer knapper (vgl. z. B. Habler, O., Pape, A., Meier, J., und Zwissler, B. (2005) [Künstliche Sauerstoffträger als Alternative zur Bluttransfusion]. Anaesthesist 54, 741-754 sowie Lowe, K. C. (2006) Blood substitutes: from chemistry to clinic. J Mat Chem 16, 4189-4196).

Zudem häufen sich die Befunde, die auf deutliche, bereits nach wenigen Tagen nachzuweisende Funktionseinbußen im konservierten Blut hinweisen (vgl. z. B. Riess, J. G. (2001) Oxygen carriers ("blood substitutes") - raison d'être, chemistry, and some physiology. Chem Rev 101, 2797-2920; Bennett-Guerrero, E., Veldman, T. H., Doctor, A., Telen, M. J., Ortel, T. L., Reid, T. S., Mulherin, M. A., Zhu, H., Buck, R. D., Califf, R. M., und McMahon, T. J. (2007) Evolution of adverse changes in stored RBCs. Proc Natl Acad Sci USA 104, 17063-17068; sowie Reynolds, J. D., Ahearn, G. S., Angelo, M., Zhang, J., Cobb, F., und Stamler, J. S. (2007) S-nitrosohemoglobin deficiency: a mechanism for loss of physiological activity in banked blood. Proc Natl Acad Sci U S A 104, 17058-17062).

Bluttransfusionen gehören zu den sehr häufig angewendeten medizinischen Prozeduren. Die Versorgung der Organe mit Sauerstoff ist die bei weitem wichtigste Einzelaufgabe des Blutes; bricht sie zusammen, stirbt der Betroffene innerhalb kürzester Zeit. Daher muß im Falle eines hohen Blutverlustes zuerst der Sauerstofftransport sichergestellt werden. Dazu werden routinemäßig Erythrozyten aus Spenderblut verwendet; diese Zellen enthalten das Protein Hämoglobin, welches Sauerstoff sehr effektiv bindet und an das Gewebe abgibt. Spenderblut hat jedoch einige erhebliche Nachteile: Es gibt nicht genug Spender, und nicht jeder Empfänger kann jedes Spenderblut bekommen (Inkompatibilität). Blut kann unbekannte infektiöse Materialien (z. B. Viren etc.) enthalten. Die Kontrolle auf gefährliche Erreger ist sehr aufwendig und teuer. Zudem ist Spenderblut nur begrenzt lagerfähig.

Eine effiziente Lösung dieses Problems ist die Entwicklung künstlicher Sauerstoffträger, welche den Einsatz von Blutkonserven überflüssig machen oder zumindest die Zahl der Bluttransfusionen deutlich vermindern.

Daher wird seit einigen Jahrzehnten an geeigneten synthetischen Ersatzstoffen geforscht - einerseits der Einsatz von modifiziertem Hämoglobin und andererseits der Einsatz perfluorierter Kohlenwasserstoffe (Perfluorcarbone), welche ein sehr gutes Bindungs- bzw. Lösungsvermögen für Sauerstoff besitzen.

Neben den Sauerstoffträgern auf der Basis von Hämoglobin-Derivaten - deren Entwicklung mit einer eigenen Problematik verbunden ist (wie z. B. knappe Ressourcen, Kontamination mit Erregern, Immunreaktionen, Durchblutungsstörungen, Toxizität etc.) - wurden und werden unter anderem Sauerstoffträger auf der Basis von perfluorierten Kohlenwasserstoffen, den sogenannten Perfluorcarbonen, entwickelt (vgl. z. B. zuvor zitierte Literatur sowie Dinkelmann, S., und Northoff, H. (2003) Artificial oxygen carriers - a critical analysis of current developments. Anästhesiologie Intensivmed Notfallmed Schmerzther 38, 47-54 sowie Spahn, D. R., und Kocian, R. (2005) Artificial O2 carriers: status in 2005. Curr Pharm Des 11, 4099-4114).

Perfluorcarbone besitzen eine hohe Löslichkeit für respiratorische Gase, wie Sauerstoff und Kohlendioxid, welche sie in Kavitäten, d. h. kleinen Hohlräumen, lösen. Sie sind chemisch weitgehend inert, farb- und geruchlos und wirken in keiner Weise korrosiv. Ihre physikalischen Eigenschaften, wie Schmelz- und Siedepunkte, variieren - wie bei den Alkanen - mit der Länge der Kette bzw. mit der Größe des Kohlenstoffgerüsts. Ihre hohe Inertheit und die damit im Zusammenhang stehende fehlende Metabolisierbarkeit sind wahrscheinlich auch der Grund dafür, daß Perfluorcarbone praktisch nicht toxisch sind. Perfluorcarbone sind extrem hydrophob, darüber hinaus aber auch lipophob. Sie werden im Stand der Technik als künstliche Sauerstoffträger - zusammen mit Emulgatoren - deshalb in Form von Emulsionen eingesetzt, wobei als Emulgatoren beispielsweise synthetische Polymere oder in jüngster Zeit Phospholipide (z. B. Lecithin, teilweise gemeinsam mit Cholesterin) verwendet werden.

Die ersten Versuche mit Perfluorcarbonen als künstliche Sauerstoffträger fanden bereits in den 1960iger Jahren statt (vgl. z. B. Clark, L. C., Jr., und Gollan, F. (1966) Survival of mammals breathing organic liquids equilibrated with oxygen at atmospheric pressure. Science 152, 1755-1756). In teils spektakulären Experimenten - in Perfluorcarbonlösungen untergetauchte Mäuse überlebten - wurde das grundsätzliche Potential dieser Verbindungen als Sauerstoffträger gezeigt. Wesentliche Probleme der ersten Generation der Perfluorcarbon-Sauerstoffträger waren die geringe Stabilität der Perfluorcarbonemulsionen sowie Nebenwirkungen, die im Zusammenhang mit dem verwendeten Emulgator standen.

Diese Probleme konnten durch Einsatz anderer Perfluorcarbone, wie 1-Bromperfluoroctan (= Perfluoroctylbromid), sowie von Lecithin als Emulgator gelöst werden. Perfluorcarbonpräparationen der neueren Generation, wie das Präparat Oxygent®, wurden erfolgreich in Tierversuchen und klinischen Studien eingesetzt (vgl. z. B. Spahn, D. R. (1999) Blood substitutes. Artificial oxygen carriers: perfluorocarbon emulsions. Crit Care 3, R93-97).

Im wesentlichen zwei ursächlich miteinander verknüpfte Probleme limitieren jedoch derzeit den weiteren klinischen Einsatz von Perfluorcarbonen als künstliche Sauerstoffträger: Einerseits ist dies eine nur relativ geringe Verweildauer der Perfluorcarbone von nur wenigen Stunden im Blutgefäßsystem; was bislang einen nur kurzfristigen Einsatz der Perfluorcarbone als Überbrückungsmaßnahme erlaubt, jedoch einen Einsatz als echte Alternative zur Bluttransfusion verhindert. Andererseits sind dies eine Störung des Immunsystems und damit unter anderem verbundene grippe-ähnliche Symptome und die Gefahr einer erhöhten Infektanfälligkeit.

Die geringe Verweildauer im Blutgefäßsystem wie auch die Störung des Immunsystems beruhen darauf, daß die Emulsionströpfchen der Perfluorcarbone von Zellen des retikuloendothelialen Systems aufgenommen werden. Diese Aufnahme geschieht zum einen aufgrund der ausgedehnten Verbreitung dieses Systems sehr schnell, und zum anderen werden die Zellen des retikuloendothelialen Systems durch die Aufnahme von Perfluorcarbonen in einigen ihrer Funktionen, wie der Bildung von Entzündungsmediatoren, aktiviert, dagegen in anderen Funktion, wie der Erregerabwehr, jedoch geschwächt.

Darüber hinaus beschreibt die WO 2007/134304 A1 eine Zusammensetzung zur kontrollierten Freigabe von Peroxiden oder Sauerstoff an eine wässrige oder nichtwässrige Umgebung, wobei die Zusammensetzung ein Kapsel- oder Beschichtungsmaterial enthält, in welches Wasserstoffperoxid, anorganische Peroxide oder Peroxidaddukte eingeschlossen sind. Durch einströmendes Wasser ins Kapselinnere oder katalytische Aktivität außerhalb der Kapsel werden die Peroxide zu Sauerstoff oder Wasserstoffperoxid umgesetzt. Das Kapselsystem ist nur für eine einmalige Abgabe von Sauerstoff bzw. Wasserstoffperoxid an das Blut oder Gewebe vorgesehen.

In bezug auf weitergehende Einzelheiten zu künstlichen Sauerstoffträgern, insbesondere als Alternative zu Bluttransfusionen, und den damit verbundenen Nachteilen und Risiken kann insbesondere verwiesen werden auf Habler, O., Pape, A., Meier, J., und Zwissler, B. (2005) [Künstliche Sauerstoffträger als Alternative zur Bluttransfusion]. Anaesthesist 54, 741-754 sowie Riess, J. G. (2001) Oxygen carriers ("blood substitutes") - raison d'être, chemistry, and some physiology. Chem Rev 101, 2797-2920.

Der vorliegenden Erfindung liegt daher die **Aufgabe** zugrunde, künstliche Sauerstoffträger, vorzugsweise auf Basis fluorierter, insbesondere perfluorierter Kohlenwasserstoffe, insbesondere auf Basis von Perfluorcarbonen, bereitzustellen, welche die zuvor geschilderten Nachteile des Standes der Technik zumindest teilweise vermeiden oder aber wenigstens abschwächen. Insbesondere sollten derartige künstliche Sauerstoffträger geeignet bzw. imstande sein, beispielsweise als Blutersatzstoff ("Blutsurrogat" bzw. "Blutsubstitut"), insbesondere bei Zuständen bei bzw. nach Blutverlusten des menschlichen oder tierischen Körpers (z. B. nach operativen Eingriffen, Unfällen, Verletzungen etc.), oder zur prophylaktischen und/oder therapeutischen Behandlung von ischämischen Zuständen oder von Zuständen nach Reperfusion (sogenanntes Tourniquet- oder Reperfusions-Syndrom), eingesetzt werden zu können.

Die Anmelderin hat nun überraschenderweise herausgefunden, daß sich die zuvor definierte Aufgabenstellung dadurch lösen läßt, daß man künstliche Sauerstoffträger auf Basis von fluorierten, insbesondere perfluorierten Kohlenwasserstoffen, vorzugsweise Perfluorcarbonen, einsetzt, wobei man die fluorierten Kohlenwasserstoffe in ein sauerstoffpermeables Kapselmaterial einschließt, insbesondere hierin einlagert oder hiervon umhüllt.

Gegenstand der vorliegenden Erfindung - gemäß einem **ersten** Aspekt der vorliegenden Erfindung - ist somit eine Dispersion von künstlichen Sauerstoffträgern nach Anspruch 1; weitere, vorteilhafte Ausgestaltungen dieses Erfindungsaspekts sind Gegenstand der diesbezüglichen Unteransprüche.

Weiterer Gegenstand der vorliegenden Erfindung - gemäß einem **zweiten** Aspekt der vorliegenden Erfindung - ist die Verwendung der erfindungsgemäßen Dispersion gemäß den Ansprüchen 9 bis 14; weitere, vorteilhafte Ausgestaltungen dieses Erfindungsaspekts sind Gegenstand der diesbezüglichen Unteransprüche.

Es versteht sich von selbst, daß nachfolgend solche Ausführungen, welche zu einem erfindungsgemäßen Aspekt gemacht werden, entsprechend auch für die anderen Erfindungsaspekte entsprechend gelten, ohne daß dies ausdrücklich vermerkt oder hierauf hingewiesen ist.

Gegenstand der vorliegenden Erfindung - gemäß einem **ersten** Aspekt der vorliegenden Erfindung - ist somit eine Dispersion von künstlichen Sauerstoffträgern, insbesondere als Blutersatzstoff, vorzugsweise zu Zwecken der Transfusion, wobei die Dispersion Kapseln mit reversibler Sauerstoffspeicherfähigkeit enthält,
wobei die Kapseln ein sauerstoffpermeables Kapselmaterial umfassen, welches fluorierte, insbesondere perfluorierte Kohlenwasserstoffe, vorzugsweise Perfluorcarbone, enthält und/oder einschließt, wobei das sauerstoffpermeable Kapselmaterial ein sauerstoffpermeables organisches Polymer umfasst oder hieraus besteht, wobei das Polymer ausgewählt ist aus der Gruppe von Poly(lactid-co-glycoliden) und Polyalkylcyanacrylaten sowie deren Mischungen,
wobei das organische Polymer frei von Sulfidbrücken ausgebildet ist und eine kohärente Struktur aufweist,
wobei in den Kapseln das Gewichtsverhältnis von sauerstoffpermeablem Kapselmaterial zu fluorierten Kohlenwasserstoffen im Bereich von 75 : 25 bis 10 : 90 liegt,
wobei die fluorierten Kohlenwasserstoffe Molekulargewichte im Bereich von 250 bis 2.000 g/mol aufweisen und flüssig ausgebildet sind,
wobei die Kapseln einen Durchmesser im Bereich von 50 bis 1.000 nm aufweisen,
wobei die Dispersion die Kapseln in volumenbezogenen Mengen von 1 bis 70 Vol.-% enthält, bezogen auf die Dispersion, wobei die Dispersion auf einen physiologischen pH-Wert eingestellt ist und eine spezifische Viskosität η im Bereich von 0,1 bis 1,8 s aufweist, bezogen auf Temperaturen im Bereich von 10 °C bis 40 °C, und wobei die Dispersion eine wässrig-basierte Dispersion ist und/oder als kontinuierliche Phase Wasser in Form einer physiologischen und/oder isotonischen Natriumchloridlösung enthält, wobei die Dispersion lagerstabil ist.

Eine Besonderheit der vorliegenden Erfindung ist somit darin zu sehen, daß die fluorierten Kohlenwasserstoffe, welche Sauerstoff reversibel zu speichern imstande sind, in verkapselter Form, insbesondere in Form von sogenannten Nanokapseln, vorliegen. Wie nachfolgend noch ausgeführt, führt dies überraschenderweise zu den nachstehend aufgeführten Vorteilen der vorliegenden Erfindung. Eine entscheidende Idee der vorliegenden Erfindung ist somit die Verwendung von perfluorcarbonhaltigen Kapseln, insbesondere Nanokapseln, als künstliche Sauerstoffträger.

Der Begriff der reversiblen Sauerstoffspeicherfähigkeit in bezug auf die erfindungsgemäß eingesetzten Kapseln, insbesondere Nanokapseln, bezeichnet insbesondere die Fähigkeit dieser Kapseln, den Sauerstoff so lange zu speichern, bis er unter physiologischen Bedingungen, insbesondere im menschlichen oder tierischen Körper, bedarfsweise wieder abgegeben bzw. freigesetzt wird, insbesondere in den betreffenden Geweben, in welchen Sauerstoffmangel bzw. ischämische Zustände vorliegen.

Der Begriff der Sauerstoffspeicherfähigkeit bezeichnet jede Art des Speicherns, beispielsweise durch physikalische und/oder chemische Bindung, Aufnahme, Einschluß, Adsorption, Lösung oder dergleichen.

In erfindungsgemäß bevorzugter Weise sind die Kapseln, insbesondere Nanokapseln, als sogenannte Kern/Hülle-Kapseln ausgebildet. Dabei bildet das sauerstoffpermeable Kapselmaterial die Kapselhülle aus, und die fluorierten Kohlenwasserstoffe sind von der sauerstoffpermeable Kapselhülle umgeben bzw. eingeschlossen, d.h. die fluorierten Kohlenwasserstoffe bilden das Innere bzw. den Kapselkern dieser Kern/Hülle-Kapseln.

Alternativ, obgleich erfindungsgemäß weniger bevorzugt, ist es jedoch auch möglich, daß die Kapseln, insbesondere Nanokapseln, als sogenannte Matrixkapseln vorliegen. Hierbei bildet das sauerstoffpermeable Kapselmaterial eine Matrix aus, und die fluorierten Kohlenwasserstoffe sind in die Matrix auf Basis dieses sauerstoffpermeablen Kapselmaterials eingelagert bzw. eingeschlossen, vorzugsweise in homogener bzw. gleichmäßiger Verteilung.

Was das sauerstoffpermeable Kapselmaterial, insbesondere die Kapselhülle der Kern/Hülle-Kapseln oder das Matrixmaterial der Matrixkapseln, anbelangt, so umfaßt dieses ein sauerstoffpermeables organisches Polymer oder besteht hieraus. Erfindungsgemäße sauerstoffpermeable organische Polymere sind Poly(lactid-co-glycolide), welche gegebenenfalls mit perfluorierten Verbindungen und/oder mit Polyalkylenglykol (insbesondere Polyethylenglykol) derivatisiert sein können, oder Polyalkylcyanacrylate, welche gegebenenfalls fluoriert sein können, sowie Mischungen dieser Polymere. Durch eine Derivatisierung der vorgenannten Polymere kann insbesondere eine verringerte Aggregationsneigung sowie bei Anwendung ein verbesserte Phagozytose erreicht werden.

Gemäß einer besonderen Ausführungsform kann das sauerstoffpermeable Kapselmaterial, insbesondere die Kapselhülle der Kern/Hülle-Kapseln oder das Matrixmaterial der Matrixkapseln, ein insbesondere sauerstoffpermeables organisches Polymer umfassen oder hieraus bestehen. Insbesondere kann das organische Polymer durch geeignete Polymerisationsverfahren, insbesondere Emulsionspolymerisation, Grenzflächenpolymerisation oder Grenzflächenfällung, vorzugsweise Grenzflächenpolymerisation oder Grenzflächenfällung, erhältlich oder hergestellt ist (z. B. Herstellung mittels Grenzflächenfällung von geeigneten Polymeren wie beispielsweise bei der Herstellung der vorgenannten Poly(lactid-co-glycolide) oder aber z. B. Herstellung mittels Grenzflächenpolymerisation beispielsweise bei der Herstellung der vorgenannten Polyalkylcyanacrylate). Erfindungsgemäß ist das organische Polymer bzw. die hieraus gebildete Kapselhülle oder Kapselmatrix frei von Sulfidbrücken, insbesondere frei von Di- oder Polysulfidbrücken, ausgebildet, da diese die Sauerstoffaufnahme und den Sauerstofftransport beeinträchtigen können. Weiterhin ist es erfindungsgemäß vorgesehen, dass das organische Polymer, insbesondere die Kapselhülle der Kern/Hülle-Kapseln oder das Matrixmaterial der Matrixkapseln, eine kohärente (d. h. eine zusammenhängende) Struktur aufweist (beispielsweise im Unterschied zu Kapseln, deren Kapselhülle aus reinen Addukten gebildet ist); auf diese Weise wird eine zuverlässige Sauerstoffspeicher- und Sauerstofftransportkapazität gewährleistet.

Gemäß einer besonderen Ausführungsform kann das sauerstoffpermeable Kapselmaterial, insbesondere die Kapselhülle der Kern/Hülle-Kapseln oder das Matrixmaterial der Matrixkapseln, vorzugsweise das insbesondere sauerstoffpermeable organische Polymer der Kapselhülle oder Matrix, eine Modifizierung, insbesondere Oberflächenmodifizierung, und/oder funktionelle Gruppen aufweisen. Die Modifizierung, insbesondere Oberflächenmodifizierung, und/oder die funktionelle Gruppen kann bzw. können dabei insbesondere ausgewählt sein aus: (i) aggregationsverhindernden funktionellen Gruppen, (ii) die Wechselwirkung mit biologischen Molekülen steuernden funktionellen Gruppen, (iii) sauren funktionellen Gruppen, (iv) Hydroxylgruppen, (v) unter physiologischem pH-Wert vorzugsweise anionischen Gruppen, insbesondere Carbonsäure-, Sulfat-, Sulfonat-, Phosphat- und Phosphonatgruppen, (vi) Polyalkylenpolyolgruppen, insbesondere Polyethylenglykolgruppen, (vii) Emulgatoren, (viii) Dispergiermitteln sowie deren Kombinationen und/oder Mischungen untereinander. Auf diese Weise können die Aggregrationseigenschaften der Kapseln gezielt gesteuert und kann eine verbesserte Dispergierbarkeit erreicht werden. Auch kann eine Verbesserung in bezug auf die Phagozytose sowie die Sauerstoffbindungs- und Sauerstoffüberragungseigenschaften erreicht werden.

In erfindungsgemäß bevorzugter Weise ist das sauerstoffpermeable Kapselmaterial, insbesondere die Kapselhülle der Kern/Hülle-Kapseln bzw. das Matrixmaterial der Matrixkapseln, außerdem CO₂-permeabel ausgebildet.

Was die Größe der Kapseln anbelangt, so weisen diese einen Durchmesser von höchstens 1.000 nm auf; auf diese Weise ist gewährleistet, daß die Kapseln die betreffenden Blutgefäße, Gewebe, Organe etc. ohne weiteres passieren können. Andererseits weisen die Kapseln einen Mindestdurchmesser von mindestens 50 nm auf, insbesondere um über ausreichende Mengen an Sauerstoffspeicherkapazität zu verfügen.

Erfindungsgemäß weisen die Kapseln einen Durchmesser im Bereich von 50 bis 1.000 nm, noch mehr bevorzugt 50 bis 975 nm, auf.

In erfindungsgemäß besonders bevorzugter Weise liegen die Kapseln in Form von Nanokapseln vor, vorzugsweise mit Kern/Hülle-Struktur. Bevorzugte Nanokapseln mit Kern/Hülle-Struktur weisen einen Durchmesser im Bereich von 50 bis 1.000 nm, noch mehr bevorzugt 50 bis 975 nm, auf.

Vorteilhafterweise liegen die erfindungsgemäß eingesetzten Kapseln in multimodaler Teilchengrößeverteilung vor. Insbesondere kommen dabei mittlere Durchmesser D50 der Kapseln im Bereich von 50 bis 1.000 nm, noch mehr bevorzugt 50 bis 950 nm, in Betracht.

Es versteht sich von selbst, daß bei allen vorstehend sowie nachfolgend noch genannten Werten und Bereichsangaben der Fachmann anwendungsbezogen oder einzelfallbedingt von den genannten Werten und Bereichen abweichen kann, ohne daß der Rahmen der vorliegenden Erfindung verlassen ist.

Was das Gewichtsverhältnis von sauerstoffpermeablem Kapselmaterial zu fluorierten Kohlenwasserstoffen in den Kapseln anbelangt, so sollte dieses höchstens 75 : 25, vorzugsweise höchstens 50 : 50, betragen. Erfindungsgemäß variiert das Gewichtsverhältnis von sauerstoffpermeablem Kapselmaterial zu fluorierten Kohlenwasserstoffen in den Kapseln im Bereich von 75 : 25 bis 10 : 90, bevorzugt 70 : 30 bis 15 : 85. Je höher der Anteil an fluorierten Kohlenwasserstoffen ist, desto größer ist die Sauerstoffspeicherfähigkeit, wobei jedoch bei zu geringen Anteilen an Kapselmaterial die Stabilität der erfindungsgemäß eingesetzten Kapseln nicht mehr gewährleistet ist, was die zuvor genannten Wertebereiche erklärt.

Im allgemeinen besitzen die erfindungsgemäß eingesetzten Kapseln eine Dichte von 1,5 bis 2,5 g/ml, insbesondere 1,8 bis 2 g/ml, vorzugsweise 1,85 bis 1,9 g/ml.

Obwohl die Entwicklung von Kapseln, insbesondere Nanokapseln, für den medizinischen Einsatz im allgemeinen seit geraumer Zeit intensiv betrieben wird und Nanokapseln in Therapie und Diagnostik bereits als Vehikel für verschiedene Arzneimittel bzw. Diagnostika genutzt werden bzw. zumindest vorgeschlagen worden sind, sind Nanokapseln, insbesondere als Vehikel für fluorierte Kohlenwasserstoffe, wie Perfluorcarbone, für den Einsatz als künstliche Sauerstoffträger, insbesondere für die Verwendung als Blutersatzstoff ("Blutsurrogat" bzw. "Blutsubstitut"), bislang noch nicht in Betracht gezogen worden. Ein derartiges Konzept konnte überraschenderweise erst im Rahmen der vorliegenden Erfindung realisiert werden.

Erst im Rahmen der vorliegenden Erfindung wurde nämlich überraschend gefunden, daß es möglich ist, Kapseln, insbesondere Nanokapseln, mit fluorierten Kohlenwasserstoffen, insbesondere Perfluorcarbonen, zu entwickeln, welche sich als künstliche Sauerstoffträger einsetzen lassen. Ein solcher Ansatz ist bislang nicht in Erwägung gezogen worden, weil die Kapselhülle die Sauerstofftransporteigenschaft dieser Stoffe negativ beeinträchtigen kann, so daß ein nur unzureichendes Sauerstoffspeicher-, -transport und -freisetzungsvermögen resultiert. Auch die Verwendung von partikulären Systemen (d. h. Kapseln) im Unterschied zu Emulsionen stand einer Umsetzung, insbesondere im Hinblick auf die erforderliche Biokompatibilität bzw. physiologische Kompatibilität, bislang entgegen.

Es wurde aber nun im Rahmen der vorliegenden Erfindung unerwartet gefunden, daß die erfindungsgemäß eingesetzten Perfluorcarbon-Nanokapseln ähnliche O₂-Transporteigenschaften wie reine Perfluorcarbon-Emulsionen besitzen, jedoch - insbesondere bei geeigneter Wahl des Kapselmaterials - mit deutlich geringerer Geschwindigkeit von den Zellen des retikuloendothelialen Systems aufgenommen werden. Auf diese Weise können in überraschender Weise die im Stand der Technik den klinischen Einsatz der Perfluorcarbone limitierenden Probleme, insbesondere die zu kurzen Aufenthaltszeiten im Gefäßsystem sowie eine Störung des Immunsystems, entscheidend vermieden bzw. signifikant verringert werden.

Was die erfindungsgemäß eingesetzten fluorierten, insbesondere perfluorierten Kohlenwasserstoffe, vorzugsweise Perfluorcarbone, anbelangt, so sind diese zur reversiblen Speicherung und/oder Aufnahme von Sauerstoff, insbesondere unter physiologischen Bedingungen, imstande bzw. besitzen ein reversibles Sauerstoffspeichervermögen, insbesondere unter physiologischen Bedingungen. Infolge der Reversibilität des Sauerstoffspeichervermögens sind die fluorierten Kohlenwasserstoffe imstande, den Sauerstoff bedarfsweise, insbesondere unter physiologischen Bedingungen, direkt am Ort des Bedarfs wieder abzugeben bzw. freizusetzen, beispielsweise in Geweben bzw. Organen ischämischen Zustands.

Üblicherweise sind die erfindungsgemäß eingesetzten fluorierten, insbesondere perfluorierten Kohlenwasserstoffe, vorzugsweise Perfluorcarbone, flüssig, insbesondere unter physiologischen Bedingungen.

Die erfindungsgemäß eingesetzten fluorierten, insbesondere perfluorierten Kohlenwasserstoffe, vorzugsweise Perfluorcarbone, besitzen Molekulargewichte im Bereich von 250 bis 2.000 g/mol, insbesondere 300 bis 1.000 g/mol, bevorzugt 400 bis 600 g/mol. Dabei sind die eingesetzten fluorierten Kohlenwasserstoffe vorzugsweise flüssig ausgebildet, bezogen auf Normalbedingungen, insbesondere Umgebungstemperaturen (z. B. 20 °C) und Atmosphärendruck.

Im allgemeinen werden als fluorierte Kohlenwasserstoffe perfluorierte Kohlenwasserstoffe, insbesondere Perfluorcarbone, eingesetzt; hierbei handelt es sich um vollständig mit Fluoratomen substituierte Kohlenstoffverbindungen. Dennoch ist es erfindungsgemäß auch möglich, fluorierte Kohlenwasserstoffe einzusetzen, welche mindestens ein von Fluor verschiedenes Halogen, vorzugsweise Brom, enthalten, wie z. B. Bromperfluoroctan.

Im allgemeinen sind die erfindungsgemäß eingesetzten fluorierten Kohlenwasserstoffe ausgewählt aus der Gruppe von Perfluoroctan, Perfluordekan, Perfluordekalin und Bromperfluoroctan sowie deren Mischungen, insbesondere Perfluoroctan, Perfluordekan und Perfluordekalin sowie deren Mischungen, vorzugsweise Perfluoroctan und/oder Perfluordekan.

Die volumenbezogene Menge an Kapseln in den erfindungsgemäßen Dispersionen kann in weiten Bereichen variieren. Die erfindungsgemäßen Dispersionen enthalten die Kapseln in volumenbezogenen Mengen von 1 bis 70 Vol.-%, insbesondere 5 bis 65 Vol.-%, vorzugsweise 10 bis 60 Vol.-%, besonders bevorzugt 20 bis 50 Vol.-%, bezogen auf die Dispersionen.

Die massenbezogene Menge der Kapseln in den erfindungsgemäßen Dispersionen kann gleichermaßen in breiten Bereichen variieren. Im allgemeinen enthalten die erfindungsgemäßen Dispersionen die Kapseln in massenbezogenen Mengen von 5 bis 80 Gew.-%, insbesondere 10 bis 75 Gew.-%, vorzugsweise 15 bis 65 Gew.-%, bezogen auf die Dispersionen.

Um eine komplikationsfreie Anwendung bei der Behandlung des menschlichen oder tierischen Körpers zu ermöglichen, sind die erfindungsgemäßen Dispersionen auf einen physiologischen pH-Wert und/oder auf eine physiologische Ionenstärke und/oder auf eine physiologische Osmolarität und/oder auf eine physiologische Ionenzusammensetzung eingestellt.

Im allgemeinen liegt der pH-Wert der erfindungsgemäßen Dispersionen im Bereich von 6,5 bis 7,9, insbesondere 7 bis 7,5, vorzugsweise 7 bis 7,45, besonders bevorzugt 7,37 bis 7,45, und entspricht somit im wesentlichen dem pH-Wert von natürlichem Blut bzw. Blutplasma.

Zur Einstellung bzw. Aufrechterhaltung des pH-Wertes kann es vorgesehen sein, einen Puffer bzw. ein Puffersystem, insbesondere einen physiologischen Puffer bzw. ein physiologisches Puffersystem, vorzugsweise auf Phosphatbasis, zuzusetzen.

Für eine komplikationsfreie Anwendung sollten die erfindungsgemäßen Dispersionen im wesentlichen eine Konsistenz und Viskosität vergleichbar der von natürlichem Blut bzw. Blutserum aufweisen. Die erfindungsgemäßen Dispersionen weisen eine spezifische Viskosität η im Bereich von 0,1 bis 1,8 s, insbesondere 0,2 bis 1,5 s, vorzugsweise 0,25 bis 1,25 s, auf, wobei die vorgenannten Viskositätswerte insbesondere auf Temperaturen im Bereich von 10 °C bis 40 °C bezogen sind.

Die erfindungsgemäßen Dispersionen sind als wäßrig-basierte Dispersion ausgebildet, d.h. die erfindungsgemäßen Dispersionen enthalten als kontinuierliche Phase (d.h. als Dispersionsmittel bzw. Dispergens) Wasser, insbesondere in Form einer vorzugsweise physiologischen und/oder isotonischen Natriumchloridlösung, bevorzugt mit einer Natriumchloridkonzentration von 0,9 Gew.-%, bezogen auf die kontinuierliche Phase.

Was die Osmolarität der erfindungsgemäßen Dispersion anbelangt, so kann diese gleichermaßen in breiten Bereichen variieren. Im allgemeinen weisen die erfindungsgemäßen Dispersionen Osmolaritäten im Bereich von 250 bis 350 mosmol/l, insbesondere 280 bis 310 mosmol/l, auf.

Des weiteren kann es vorgesehen sein, daß die erfindungsgemäßen Dispersionen au-βerdem übliche Hilfs- und/oder Zusatzstoffe (Additive) enthalten, welche insbesondere ausgewählt sein können aus der Gruppe von Dispergierhilfsmitteln, Emulgatoren, Streckmitteln, Bindemitteln, Netzmitteln, Stabilisierungsmitteln sowie deren Mischungen. Als Emulgatoren können beispielsweise Lecithin und/oder Cholesterin und/oder Derivate von Gallensäuren und/oder Salze der vorgenannten Verbindungen (z. B. Cholate, wie Alkalicholate, vorzugsweise Natriumcholat) zum Einsatz kommen. Jedoch sollte die Menge an solchen Hilfs- und/oder Zusatzstoffen (Additiven), insbesondere die Menge an Dispergierhilfsmitteln und/oder Emulgatoren (z. B. Natriumcholat), in den erfindungsgemäßen Dispersionen vorteilhafterweise eine Konzentration von 50 µmol/l, insbesondere 40 µmol/l, vorzugsweise 35 µmol/l, nicht überschreiten. Höhere Konzentration dagegen (z. B. höhere Emulgatorkonzentration) können zytotoxischen Einfluß haben und sollten daher vermieden werden.

Die vorliegende Erfindung stellt somit künstliche Sauerstoffträger auf Basis fluorierter Kohlenwasserstoffe, insbesondere auf Basis von Perfluorcarbonen, in Form von Dispersionen der zuvor definierten Kapseln aus sauerstoffpermeablem Kapselmaterial und hierin enthaltenen bzw. hiervon eingeschlossenen fluorierten Kohlenwasserstoffen als eigentlichen Sauerstoffträgern (d. h. unter physiologischen Bedingungen reversiblen Sauserstoffspeichern) bereit, welche die zuvor geschilderten Nachteile des Standes der Technik zumindest teilweise vermeiden bzw. abschwächen.

Insbesondere sind die künstlichen Sauerstoffträger bzw. die sie enthaltenden Dispersionen geeignet bzw. imstande, beispielsweise als Blutersatzstoff ("Blutsurrogat" bzw. "Blutsubstitut"), insbesondere bei Zuständen bei bzw. nach Blutverlusten des menschlichen oder tierischen Körpers (z. B. nach operativen Eingriffen, Unfällen, Verletzungen etc.), oder zur prophylaktischen und/oder therapeutischen Behandlung von ischämischen Zuständen oder von Zuständen nach Reperfusion (sogenanntes Tourniquet- oder Reperfusions-Syndrom), eingesetzt werden zu können.

Im Rahmen der vorliegenden Erfindung können Perfluorcarbon-Nanokapseln mit unterschiedlichen Kapselmaterialien und Oberflächeneigenschaften und mit verschiedenen Perfluorcarbonen hergestellt werden.

Die Herstellung der erfindungsgemäß einzusetzenden Kapseln, insbesondere Perfluorcarbon-(Nano)Kapseln, kann in dem Fachmann an sich bekannter Weise erfolgen. Dies kann beispielsweise durch in-situ-Polymerisation geeigneter Ausgangsmonomere, welche dann das Kapselmaterial bzw. die Kapselwandungen ausbilden, in Gegenwart der zu verkapselnden Perfluorcarbone erfolgen (z. B. mittels geeigneter Polymerisationsverfahren, insbesondere Emulsionspolymerisation, Grenzflächenpolymerisation etc.).

Die Perfluorcarbon-(Nano)Kapseln und die sie enthaltenden Dispersionen bzw. Arzneimittel werden insbesondere mit mikroskopischen und spektroskopischen Methoden charakterisiert, und zudem werden ihre Biokompatibilität, ihre Aufnahme in Zellen und ihre Fähigkeit zum Sauerstofftransport bzw. zur Sauerstoffversorgung in einfachen biologischen Systemen unter Einsatz der Laser-Scanning-Mikroskopie und der Intravital-Mikroskopie sowie auch in komplexeren Systemen bis hin zu Tierversuchen bestätigt.

Im Rahmen der vorliegenden Erfindung kommen vorzugsweise Kapseln, insbesondere Nanokapseln, mit gegebenenfalls mit perfluorierten Verbindungen und/oder mit Polyethylenglykol modifizierten Poly(lactid-co-glycoliden) oder mit gegebenenfalls fluorierten, insbesondere perfluorierten Polyalkylcyanacrylaten als Kapselmaterial, insbesondere Hülle, und Perfluoroctan, Perfluordekalin bzw. Perfluordekan als Kapselinneres bzw. Kapselkern zum Einsatz, welche im menschlichen oder tierischen Körper als künstliche Sauerstoffträger eingesetzt werden können. Damit wird eine entscheidende Grundlage als Alternative zur Bluttransfusion gelegt.

Die Kapselhülle der erfindungsgemäß eingesetzten Perfluorcarbon-(Nano)Kapseln auf Basis von gegebenenfalls mit perfluorierten Verbindungen modifiziertem Poly(lactid-co-glycolid) oder auf Basis von gegebenenfalls fluoriertem Polyalkylcyanacrylat etc. bestimmt insbesondere die biologischen Eigenschaften, während der Kapselkern mit bzw. aus Perfluorcarbonen, wie z. B. Perfluordekan, Perfluordekalin, Perfluoroctan etc., der eigentliche Sauerstoffträger ist.

Die erfindungsgemäß bereitgestellten Perfluorcarbon-(Nano)Kapseln bzw. die sie enthaltenden Dispersionen bzw. Pharmazeutika (Arzneimittel) ermöglichen sehr vielfältige Anwendungsmöglichkeiten. Beispielsweise lassen sie sich bei allen Zuständen des Sauerstoffmangels im menschlichen oder tierischen Organismus einsetzen. Beispiele hierfür sind Schädigungen von Zellen, Geweben und Organen durch Ischämie und Reperfusion; typische Schädigungen durch Ischämie und Reperfusion sind Herzinfarkt, Schlaganfall, Schockzustände nach starken Blutverlusten sowie die Schädigung von Spenderorganen bei der Transplantation. Mechanismen, die in diesem Zusammenhang untersucht worden sind, sind die Störung der zellulären Ionenhomöostase in der Phase der Ischämie (d. h. der Phase des Sauerstoffmangels aufgrund der Unterbrechung der Durchblutung) sowie die Beteiligung von reaktiven Sauerstoff- und Stickstoffspezies an der Schädigung in der Phase der Reperfusion (d. h. der Wiederdurchblutung und damit der Wiederversorgung mit Sauerstoff, ein Vorgang, der mit einer eigenständigen Schädigungskomponente verbunden ist). Die erfindungsgemäß bereitgestellten Perfluorcarbon-(Nano)Kapseln bzw. die sie enthaltenden Dispersionen bzw. Pharmazeutika ermöglichen somit beispielsweise den Einsatz als Protektionslösung bzw. Protektionsdispersion zum Schutz von Organen bei der Transplantation (wie z. B. Herz, Leber, Niere, Lunge, Pankreas, Darm etc.).

Im Rahmen der vorliegenden Erfindung kommen einheitliche und lagerstabile Kapseldispersionen von Perfluorcarbon-(Nano)Partikeln zum Einsatz. In an sich bekannter Weise kann die Herstellung der erfindungsgemäß einzusetzenden Kapseln beispielsweise über die zwischenzeitliche Bildung einer Miniemulsion realisiert werden, die zu besonders stabilen Dispersionen mit engen Größenverteilungen führt. Die Abhängigkeiten zwischen den Präparationsparametern und den wichtigsten Eigenschaften der Kapseldispersionen wurden in systematischen Versuchsreihen unter Anwendung statistischer Versuchsplanungen herausgearbeitet. In Verbindung mit der Entwicklung der Präparationsverfahren wurden darüber hinaus neue Charakterisierungsmethoden entwickelt, die insbesondere auf dem Einsatz der kernmagnetischen Resonanzspektroskopie beruhen.

Die Wahl des Kapselhüllenmaterials kann insbesondere nach folgenden Kriterien erfolgen: Realisierbarkeit und Einfachheit der Herstellung, insbesondere im Hinblick auf große Mengen, Stabilität bei Langzeitlagerung, Durchlässigkeit für O₂ und/oder CO₂, keine bestehenden Toxizitäten, biologische Abbaubarkeit, Halbwertszeit im Blutgefäßsystem, Möglichkeiten der weiteren Oberflächenmodifizierung etc.

Gemäß einer erfindungsgemäß bevorzugten Ausführungsform kann die Kapselhülle der Perfluorcarbon-Nanokapseln insbesondere aus gegebenenfalls mit perfluorierten Verbindungen modifiziertem Poly(lactid-co-glycolid) oder aus gegebenenfalls fluoriertem Polyalkylcyanacrylat gebildet werden. Im Fall der Verwendung von gegebenenfalls mit perfluorierten Verbindungen modifiziertem Poly(lactid-co-glycolid) kann das Lactid/Glycolid-Verhältnis variieren (z. B. im Bereich von 25 : 75 bis 75 : 25). Als Perfluorcarbone können in erfindungsgemäß bevorzugter Weise Perfluordekan, Pefluordekalin und Perfluoroctan zum Einsatz kommen.

Erfindungsgemäß bevorzugt eingesetzte Poly(lactid-co-glycolid)- Perfluorcarbon-(Nano)Kapseln können beispielsweise wie folgt hergestellt werden: Das Poly(lactid-co-glycolid) stellt ein Polymer dar, welches sequentiell randomisiert aus Lactyl-Einheiten (-CH(CH₃)-CO-O-) und Glycolyl-Einheiten (-CH₂-CO-O-) in variablen Mengenverhältnissen aufgebaut ist (vgl. z. B. Wang, L., Venkatraman, S., Gan, L. H., und Kleiner, L. (2005), Structure formation in injectable poly(lactide-co-glycolide) depots. II. Nature of the gel. J Biomed Mater Res B Appl Biomater 72, 215-222). Die verschiedenen Poly(lactid-co-glycolid)-Polymere unterscheiden sich einerseits im Gehalt der Lactyl-Einheiten (z. B. 50 % bis 85 %), andererseits bei gegebenem Lactyl-Gehalt durch eine unterschiedliche Viskosität. Alle Poly(lactid-co-glycolid)-Polymere werden in Säugern letztendlich zu Wasser und CO₂ abgebaut. Sie stellen damit unter physiologischen Gesichtspunkten unbedenkliche Produkte dar und werden als solche seit fast vierzig Jahren in der Pharmakologie verwendet. Die Wände dieser Kapseln sind aufgrund ihrer Porosität gut durchlässig für O₂ und CO₂. Die Einkapselung erfolgt ohne jeglichen Verlust an Funktionalität. Die Herstellung der Kapseln kann beispielsweise analog zu dem Herstellungsverfahren erfolgen, wie es beispielsweise beschrieben ist bei Pisani, E., Tsapis, N., Paris, J., Nicolas, V., Cattel, L., und Fattal, E. (2006) Polymeric nano/microcapsules of liquid Perfluorcarbons for ultrasonic imaging: physical characterization. Langmuir 22, 4397-4402. Das dort beschriebene Verfahren kann erfindungsgemäß zur Einkapselung von Perfluorcarbonen entsprechend eingesetzt werden, insbesondere um Nanokapseln als Sauerstoffträger beispielsweise mit den Kernen Perfluoroctan, Perfluordekalin oder Perfluordekan herzustellen. Das Poly(lactid-co-glycolid) kann gegebenenfalls mit perfluorierten Verbindungen modifiziert bzw. derivatisiert werden.

Erfindungsgemäß gleichermaßen bevorzugt sind gegebenenfalls fluorierte Polyalkylcyanacrylat-Perfluorcarbon-(Nano)Kapseln. Alkylcyanacrylat bietet als reaktives Monomer den Vorteil, daß es unter sehr schonenden Bedingungen an der Grenzfläche einer Öl-in-Wasser-Emulsion auspolymerisieren und dabei Kapseln bilden kann (vgl. z. B. Couvreur, P., Kante, B., Roland, M., Guiot, P., Bauduin, P., und Speiser, P. (1979) Polycyanacrylate nanocapsules as potential lysosomotropic carriers: preparation, morphological and sorptive properties. J Pharm Pharmacol 31, 331-332 sowie Florence, A. T., Whateley, T. L., und Wood, D. A. (1979) Potentially biodegradable microcapsules with poly(alkyl 2-cyanoacrylate)membranes. J Pharm Pharmacol 31, 422-42). Die anionische Polymerisation kann dabei durch eine gezielte Änderung des pH-Werts der wäßrigen Phase gestartet werden und zu einer physikalisch vernetzten, aber relativ porösen und gasdurchlässigen Polymerhülle führen. Die Präparation von Polyalkylcyanacrylat-(Nano)Kapseln ist inzwischen in mehreren Varianten beschrieben und auch in größerem Maßstab durchführbar (vgl. z. B. Wohlgemuth, M., Machtle, W., und Mayer, C. (2000) Improved preparation and physical studies of polybutylcyanoacrylate nanocapsules. J Microencapsul 17, 437-448; Mayer, C. (2005) Nanocapsules as drug delivery systems. Int J Artif Organs 28, 1163-1171; Altinbas, N., Fehmer, C., Terheiden, A., Shukla, A., Rehage, H., und Mayer, C. (2006) Alkylcyanoacrylate nanocapsules prepared from miniemulsions: a comparison with the conventional approach. J Microencapsul 23, 567-581; Al Khouri Fallouh, N., Roblet-Treupel, L., Fessi, H., Devissaguet, J. P., und Puisieux, F. (1986) Development of a new process for the manufacture of polyisobutylcyanoacrylate nanocapsules. Int J Pharm 28, 125-132; Lescure, F., Zimmer, C., Roy, D., und Couvreur, P. (1992) Optimization of polyalkylcyanoacrylate nanoparticle preparation - Influence of sulfur-dioxide and pH on nanoparticle characteristics. J Colloid Interface Sci 154, 77-86). Im Fall der Herstellung von fluorierten Polyalkylcyanacrylat-Nanokapseln kann insbesondere derart vorgegangen werden, daß Alkylcyanacrylat (z. B. Ethylcyanacrylat) zunächst durch Umesterung zu fluoriertem Alkylcyanacrylat umgesetzt wird, welches dann beispielsweise durch Grenzflächenpolymerisation um dispergierte Tröpfchen des Perfluorcarbons (z. B. Perfluordekalin) zu Kapseln aus fluorierter Alkylcyanacrylatkapselhülle mit hierin enthaltendem Perfluorcarbonkapselkern umgesetzt wird.

Die erfindungsgemäß eingesetzten Kapseln sind mechanisch und thermisch stabil und können sowohl als Dispersion als auch in gefriergetrocknetem Zustand nahezu unbegrenzt gelagert werden. Kleinere Moleküle, wie Ethanol oder Benzol, unterliegen einem raschen Austausch durch die Kapselhülle, die Halbwertszeit im eingekapselten Zustand beträgt dabei nur wenige Millisekunden. Die Durchmesser der Kapseln liegen im Bereich von Nanometern bis Mikrometern (z. B. zwischen 100 und 500 nm) und sind über die Randbedingungen der Präparation steuerbar. Das Polymer ist biologisch abbaubar, die entstehenden Abbauprodukte sind von keiner bzw. allenfalls äußerst geringer Toxizität. Polyalkylcyanacrylat-Nanokapseln sind damit vielseitige und anpaßbare Trägersysteme für die vorliegende Erfindung. Dispersionen von Polyalkylcyanacrylat-Nanokapseln können mittels amphiphiler BlockCopolymere stabilisiert werden. Um höhere Zykluszeiten im lebenden Organismus zu erzielen, kann auch eine Stabilisierung mit Chitosan, welche zu positiv geladenen Kapseln führt, erfolgen. Der besondere Vorteil der Polyalkylcyanacrylat-Nanokapseln gegenüber anderen biologisch abbaubaren Systemen liegt vor allem in der relativ langen Lebensdauer der Kapseln unter physiologischen Bedingungen.

Weiterer Gegenstand der vorliegenden Erfindung - gemäß einem **zweiten** As-pekt der vorliegenden Erfindung - ist die Verwendung der zuvor beschriebenen Dispersionen nach der vorliegenden Erfindung zur prophylaktischen und/oder therapeutischen Behandlung von Sauerstoffmangelzuständen des menschlichen oder tierischen Körpers bzw. zur Herstellung eines Arzneimittels zur prophylaktischen und/oder therapeutischen Behandlung von Sauerstoffmangelzuständen des menschlichen oder tierischen Körpers.

Der Begriff des Sauerstoffmangelzustandes des menschlichen oder tierischen Körpers, wie er im Rahmen der vorliegenden Erfindung verwendet wird, bezeichnet insbesondere ischämische und hypoxische Zustände des menschlichen oder tierischen Körpers bzw. bestimmter Gewebe oder Organe, wobei im Rahmen der vorliegenden Erfindung der Begriff der Ischämie insbesondere eine Minderdurchblutung oder einen Durchblutungsausfall eines Gewebes bzw. eines Organs bezeichnet (z. B. Zustand nach Blutverlust, Blutung etc.) und mit einem Sauerstoffmangel in dem betroffenen Gebiet einhergeht (wodurch wiederum der zelluläre Stoffwechsel behindert oder sogar letztendlich zum Erliegen kommt), während der begriff der Hypoxie im Rahmen der vorliegenden Erfindung insbesondere speziell zur Bezeichnung des Sauerstoffmangels im Gewebe verwendet wird (z. B. Zustand nach Gasvergiftung, wie z. B. Kohlenmonoxidvergiftung etc.) (wobei das vollständige Fehlen von Sauerstoff auch als Anoxie bezeichnet wird).

Weiterer Gegenstand der vorliegenden Erfindung ist die Verwendung der zuvor beschriebenen erfindungsgemäßen Dispersionen als Blutersatzstoffe, insbesondere zu Transfusionszwecken.

Beispielsweise können die erfindungsgemäßen Dispersionen zur prophylaktischen und/oder therapeutischen Behandlung von Zuständen des menschlichen oder tierischen Körpers bei bzw. nach Blutverlust, insbesondere operativen Eingriffen, Unfällen, Verletzungen oder dergleichen, verwendet werden.

Weiterhin können die erfindungsgemäßen Dispersionen gleichermaßen zur prophylaktischen und/oder therapeutischen Behandlung von ischämischen Zuständen oder von Zuständen nach Reperfusion, insbesondere des Tourniquet-Syndroms (Reperfusions-Syndroms), bzw. zur Herstellung eines Arzneimittels zur prophylaktischen und/oder therapeutischen Behandlung von ischämischen Zuständen oder von Zuständen nach Reperfusion, insbesondere des Tourniquet-Syndroms (Reperfusions-Syndroms), verwendet werden.

Des weiteren können die erfindungsgemäßen Dispersionen auch zur Protektion von Organen im Rahmen von Transplantationen eingesetzt werden. Auf diese Weise lassen sich beispielsweise die entnommenen bzw. zu transplantierenden Organe bis zum Zeitpunkt der Implantation beim Organempfänger wirksam schützen (z. B. durch Perfusion mit der erfindungsgemäßen Dispersion).

Weiterhin können die erfindungsgemäßen Dispersionen auch zur prophylaktischen und/oder therapeutischen Behandlung von Gasbläschenbildung im Blut oder von Gasembolien des menschlichen oder tierischen Körpers bzw. zur Herstellung eines Arzneimittels zur prophylaktischen und/oder therapeutischen Behandlung von Gasbläschenbildungen im Blut oder von Gasembolien des menschlichen oder tierischen Körpers eingesetzt werden (z. B. zur Behandlung von Gasembolien, wie sie nach unsachgemäßen Tauchgängen im Tauchsportbereich auftreten können). Hier kommen insbesondere die guten Gaslöseeigenschaften (z. B. für Sauerstoff, Kohlendioxid, Stickstoff etc.) der erfindungsgemäßen Dispersionen bzw. der in ihnen enthaltenen Kapseln zum Tragen.

Des weiteren können die erfindungsgemäßen Dispersionen auch zur Anwendung bei Herz/Lungen-Maschinen, insbesondere zur prophylaktischen und/oder therapeutischen Behandlung von Gasembolien des menschlichen oder tierischen Körpers bzw. zur Herstellung eines Arzneimittels zur prophylaktischen und/oder therapeutischen Behandlung von Gasembolien des menschlichen oder tierischen Körpers, eingesetzt werden. Auf diese Weise können beispielsweise Hirnschäden infolge von Sauerstoffmangelzuständen effizient verhindert werden.

Gleichermaßen können die erfindungsgemäßen Dispersionen zur prophylaktischen und/oder therapeutischen Behandlung von Gasvergiftungen oder Rauchvergiftungen des menschlichen oder tierischen Körpers bzw. zur Herstellung eines Arzneimittels zur prophylaktischen und/oder therapeutischen Behandlung von Gasvergiftungen oder Rauchvergiftungen des menschlichen oder tierischen Körpers eingesetzt werden (z. B. bei Rauchvergiftungen infolge eines Brandes oder aber bei Gasvergiftungen, wie z. B. Gasvergiftungen mit Kohlenmonoxid, Cyanwasserstoff etc.).

Bei der vorstehend genannten und nachfolgend noch beschriebenen erfindungsgemäßen Verwendungen können die erfindungsgemäßen Dispersionen insbesondere in Mengen von 5 bis 15.000 ml, insbesondere 5 bis 10.000 ml, vorzugsweise 10 bis 3.000 ml, je Einzelgabe appliziert werden, vorzugsweise mittels Transfusion.

Mit anderen Worten beschreibt die vorliegende Erfindung die Verwendung von fluorierten, insbesondere perfluorierten Kohlenwasserstoffen, vorzugsweise Perfluorcarbonen, als künstliche Sauerstoffträger zur prophylaktischen und/oder therapeutischen Behandlung des menschlichen oder tierischen Körpers bzw. zur Herstellung eines Arzneimittels zur prophylaktischen und/oder therapeutischen Behandlung des menschlichen oder tierischen Körpers, insbesondere als Blutersatzstoff, vorzugsweise zu Zwecken der Transfusion, wobei die fluorierten Kohlenwasserstoffe in Form von Kapseln mit reversibler Sauerstoffspeicherfähigkeit appliziert werden, wobei die Kapseln ein sauerstoffpermeables Kapselmaterial umfassen, welches die fluorierten Kohlenwasserstoffe enthält bzw. einschließt. Insbesondere können die Kapseln in Form einer Dispersion, wie zuvor beschrieben, appliziert bzw. eingesetzt werden. Insbesondere können die zuvor beschriebenen Kapseln zur prophylaktischen und/oder therapeutischen Behandlung von Sauerstoffmangelzuständen des menschlichen oder tierischen Körpers bzw. zur Herstellung eines Arzneimittels zur prophylaktischen und/oder therapeutischen Behandlung von Sauerstoffmangelzuständen des menschlichen oder tierischen Körpers verwendet werden können. Insbesondere lassen sich mit den zuvor beschriebenen Kapselsystemen Zustände des menschlichen oder tierischen Körpers bei und/oder nach Blutverlust, insbesondere operativen Eingriffen, Unfällen, Verletzungen oder dergleichen, prophylaktisch und/oder therapeutisch behandeln. Gleichermaßen kommt eine Verwendung der zuvor beschriebenen Kapseln zur prophylaktischen und/oder therapeutischen Behandlung von ischämischen Zuständen oder von Zuständen nach Reperfusion, insbesondere des Tourniquet-Syndroms (Reperfusions-Syndroms), in Betracht. Ebenfalls in Betracht kommt die Verwendung zum Schutz von Organen im Rahmen der Transplantation, wie zuvor beschrieben. Auch kommt eine Verwendung der zuvor beschriebenen Kapseln zur prophylaktischen und/oder therapeutischen Behandlung von Gasbläschenbildung im Blut oder von Gasembolien des menschlichen oder tierischen Körpers bzw. zur Herstellung eines Arzneimittels zur prophylaktischen und/oder therapeutischen Behandlung von Gasbläschenbildungen im Blut oder von Gasembolien des menschlichen oder tierischen Körpers in Betracht. Auch erfaßt ist eine Verwendung der zuvor beschriebenen Kapseln zur Anwendung bei Herz/Lungen-Maschinen, insbesondere zur prophylaktischen und/oder therapeutischen Behandlung von Gasembolien des menschlichen oder tierischen Körpers bzw. zur Herstellung eines Arzneimittels zur prophylaktischen und/oder therapeutischen Behandlung von Gasembolien des menschlichen oder tierischen Körpers. Schließlich kommt auch eine Verwendung der zuvor beschriebenen Kapseln zur prophylaktischen und/oder therapeutischen Behandlung von Gasvergiftungen oder Rauchvergiftungen des menschlichen oder tierischen Körpers bzw. zur Herstellung eines Arzneimittels zur prophylaktischen und/oder therapeutischen Behandlung von Gasvergiftungen oder Rauchvergiftungen des menschlichen oder tierischen Körpers in Betracht.

Gleichermaßen beschreibt die vorliegende Erfindung die Verwendung von Kapseln, insbesondere Nanokapseln, mit einem sauerstoffpermeablen Kapselmaterial und hierin enthaltenen und/oder hiervon umgebenen fluorierten, insbesondere perfluorierten Kohlenwasserstoffen, vorzugsweise Perfluorcarbonen, als künstliche Sauerstoffträger, insbesondere als Blutersatzstoff, insbesondere für die vorgenannten Einsatzzwecke und Therapien (z. B. zur Protektion von Organen bei der Transplantation bzw. zum Schutz von zu transplantierenden Organen, insbesondere im Zeitraum zwischen Organentnahme und Implantation etc.).

Für weitergehende Einzelheiten zu dem zweiten Erfindungsaspekt der vorliegenden Erfindung kann zur Vermeidung unnötiger Wiederholungen auf die vorstehenden Ausführungen zu dem ersten Erfindungsaspekt verwiesen werden, welche in bezug auf die übrigen Erfindungsaspekte entsprechend gelten.

Weitere Ausgestaltungen, Abwandlungen und Variationen der vorliegenden Erfindung sind für den Fachmann beim Lesen der Beschreibung ohne weiteres erkennbar und realisierbar, ohne daß er dabei den Rahmen der vorliegenden Erfindung verläßt.

Die nachfolgenden Ausführungsbeispiele dienen lediglich zur Veranschaulichung der vorliegenden Erfindung, ohne die vorliegende Erfindung jedoch hierauf zu beschränken.

### AUSFÜHRUNGSBEISPIELE:

### Herstellung erfindungsgemäß einsetzbarer Perfluorcarbon-(Nano)Kapseln

### Vorschrift zur Synthese von Poly(D,L-lactid-co-glycolid)-Kapseln mit einem Durchmesser von (2,5 ± 1,5) µm und anderer erfindungsgemäßer Kapselsyste-me

Poly(D,L-lactid-co-glycolid) (100 mg) mit der inneren Dichte ca. 0,45 bis 0,6 g/cm³ und Fluordekalin (100 µl) werden bei Raumtemperatur in Dichlormethan (6 ml) gelöst. Natriumcholat (1,5 %) wird in destilliertem Wasser gelöst, und diese Lösung wird anschließend auf 4 °C gekühlt. Die Dichlormethanlösung (6 ml) wird dann mit der kalten wäßrigen Natriumcholatlösung (20 ml) unter starkem Rühren in einem Eisbad gemischt. Zum Rühren wird ein Ultraturrax mit einem Verteilungsadapter verwendet (Ultraturrax T25/ SN-25-10G der Firma IKA, Rührgeschwindigkeit 13.500 U/min, Rührdauer 2 Minuten). Zur Visualisierung und Charakterisierung können auch Kapseln hergestellt werden, deren Kapselmaterial mit dem Fluoreszenzfarbstoff Nilrot angereichert ist. Diese Kapseln werden in analoger Weise hergestellt, indem 150 µl einer konzentrierten Nilrot-Lösung (0,057 mg/ml in Dichlormethan) zu der Dichlormethanlösung (6 ml), welche das Poly(D,L-lactid-co-glycolid) und das Fluordekalin enthält, pipettiert werden. In jedem Fall wird nach dem Rühren die Dichlormethanphase entfernt, indem die zweiphasige Reaktionsmischung (ca. 26 ml) mit einem kleinen Sauerstoffgasflux (0,1 bis 100 ml/min) solange gespült wird, bis die organische Phase vollständig ausgetrieben ist. Die Kapseln sedimentieren langsam (t_{1/2} etwa 6 Stunden) in der hochkonzentrierten Cholatlösung und können so abgetrennt bzw. in 0,9%iger NaCl-Lösung aufgenommen werden bzw. in erfindungsgemäße Dispersionen zur bestimmungsgemäßen Verwendung ("Blutsurrogat" bzw. "Blutsubstitut") überführt (Anwendungsmengen: 5 bis 15.000 ml, insbesondere 5 bis 10.000 ml, vorzugsweise 10 bis 3.000 ml).

In weiteren Synthesen werden erfindungsgemäß einsetzbare Perfluorcarbon-(Nano)Kapseln mit variablen Verhältnissen im Poly(D,L-lactid-co-glycolid) im Bereich von 25 : 75 bis 75 : 25 und mit variablen inneren Dichten der Kapseln von 0,16 bis 1,3 dl/g hergestellt.

In entsprechender Weise werden außerdem erfindungsgemäß einsetzbare Perfluorcarbon-(Nano)Kapseln mit einer mit perfluorierten Verbindungen derivatisierten Kapselhülle auf Basis von Poly(D,L-lactid-co-glycolid) sowie Perfluorcarbon-(Nano)Kapseln mit einer mit Polyethylenglykol derivatisierten Kapselhülle auf Basis von Poly(D,L-lactid-co-glycolid) hergestellt.

Bei der Synthese von modifizierten Poly(D,L-lactid-co-glycolid)-Kapseln z. B. mit einem Durchmesser von (2,5 ± 1,5) µM kann beispielsweise wie folgt vorgegangen werden: Poly(D,L-lactid-co-glycolid) (50 : 50, 92 mg) mit terminalen Carboxylgruppen und der inneren Dichte von 0,45 bis 0,6 dl/g wird bei Raumtemperatur in Dichlormethan (3 ml) gelöst. Ein zweites Poly(D,L-lactid-co-glycolid) (50 : 50, 8 mg), das mit einem 5.000 Dalton Polyethylenglkol ein 85 : 15-Copolymer bildet, wird ebenfalls bei Raumtemperatur in Dichlormethan (3 ml) gelöst. Nach dem Lösungsprozeß der Polymere werden die beiden Lösungen vereinigt. Anschließend wird Fluordekalin (100 µl) bei Raumtemperatur in dieser Dichlormethanlösung (6 ml) gelöst. Natriumcholat (1,5 %) wird in destilliertem Wasser gelöst, und diese Lösung wird anschließend auf 4 °C gekühlt. Die Dichlormethanlösung (6 ml) wird dann mit der kalten wäßrigen Natriumcholatlösung (20 ml) unter starkem Rühren in einem Eisbad gemischt. Zum Rühren wird ein Ultraturrax mit einem Verteilungsadapter verwendet (z. B. Ultraturrax T25/ S-25KV-25G-IL der Fa. IKA, Rührgeschwindigkeit 6000 U/min, Rührdauer 2 Minuten). Zur Visualisierung und Charakterisierung können auch Kapseln hergestellt werden, deren Kapselmaterial mit dem Fluoreszenzfarbstoff Nilrot angereichert ist. Diese Kapseln werden in analoger Weise hergestellt, indem 150 µl einer konzentrierten Nilrotlösung (0,057 mg/ml in Dichlormethan) zu der Poly(D,L-lactid-co-glycolid)/Fluordekalin enthaltenden Dichlormethanlösung (6 ml) pipettiert werden. In jedem Fall wird nach dem Rühren die Dichlormethanphase entfernt, indem die zweiphasige Reaktionsmischung (ca. 26 ml) mit einem kleinen Sauerstoffgasflux (0,1 bis 1 ml/min) solange gespült wird, bis die organische Phase vollständig ausgetrieben worden ist. Die Kapseln sedimentieren langsam (t_{1/2} etwa 6 Stunden) in der hochkonzentrierten Cholatlösung und können so abgetrennt bzw. in 0,9%iger NaCl-Lösung aufgenommen werden. Durch die Derivatisierung der Polymere kann insbesondere eine Verbesserung in bezug auf Aggregationsvermeidung und Phagozytose erzielt werden.

Des weiteren werden erfindungsgemäß einsetzbare Perfluorcarbon-Nanokapseln mit einer einer Kapselhülle auf Basis von Alkylcyanacrylat oder auf Basis von Perfluoralkylcyanacrylat hergestellt.

### Physikalisch-chemische Charakterisierung der erfindungsgemäß einsetzbaren Perfluorcarbon-(Nano)Kapseln bzw. der sie enthaltenden Dispersionen

Die erfindungsgemäß einsetzbaren Perfluorcarbon-Nanokapseln werden in ihrer Größe, ihrer homogenen Struktur, ihrem Aufbau und ihrer Zusammensetzung durch folgende Verfahren (Methoden) charakterisiert:

### Konfokale Laser-Scanning-Mikroskopie (LSM)

Die konfokale Laser-Scanning-Mikroskopie (LSM) wird zur Bestimmung der Größe und zum Nachweis der homogenen Struktur der erfindungsgemäß einsetzbaren Perfluorcarbon-Nanokapseln verwendet. Hierfür werden die Nanokapseln mit dem Fluoreszenzfarbstoff Nilrot (9-Diethylamino-5H-benzo[α]phenoxazin-5-on) gefärbt, der sich aufgrund seiner lipophilen Eigenschaften in der Kapselwand anreichert. Die LSM-Messungen werden unter Verwendung eines Helium/Neon-Lasers und hochauflösender Objektive durchgeführt. Aliquots der gefärbten und mit einem Puffer physiologischer Zusammensetzung verdünnten Perfluorcarbon-Nanokapseln werden auf die Oberfläche von mit Poly-L-Lysin behandelten Deckgläschen in modifizierten Pentz-Kammern aufgebracht und die Fluoreszenz in verschiedenen Fokusebenen der Flüssigkeitssäule dargestellt. Aufgrund der sehr geringen optischen Schichtdicke des konfokalen Systems sind somit begrenzt auch räumliche Darstellungen der Perfluorcarbon-Nanokapseln möglich. Ihre Größe und Homogenität wird basierend auf den detektierten Signalen mittels der entsprechenden Software *"Physiology Evaluation"* des LSM 510-Systems quantifiziert.

### Lichtmikroskopische Beobachtung der dispergierten Kapseln im Dunkelfeld

Die Laser Scanning-Mikroskopie ist nur eingeschränkt für die Größenbestimmung von Nanokapseln mit einem Durchmesser kleiner 1 µm geeignet. Deshalb kommt als weiteres Verfahren die lichtmikroskopische Beobachtung der dispergierten Kapseln im Dunkelfeld zum Einsatz. Die lichtmikroskopische Beobachtung der dispergierten Kapseln im Dunkelfeld dient der Bestimmung der Brown'schen Teilchenbewegung. Dabei werden die Bewegungen individueller Teilchen durch automatische Bildauswertung einer in Echtzeit erstellten Videodatei ausgewertet und zu einem Kapselgrößenhistogramm verarbeitet.

### Rastersondenmikroskopie (AFM)

Mit der Rastersondenmikroskopie wird eine Methode angewendet, die nicht nur die Abbildung der Kapseln, sondern auch die Bestimmung mechanischer Eigenschaften ermöglicht. In einem Druckversuch wird ein Kraft-Weg-Diagramm aufgezeichnet, das wichtige Daten über die mechanische Festigkeit, die reversible Verformbarkeit und die Elastizität der Kapseln liefert. Darüber hinaus erlaubt die Art der Faltung der Kapselhülle nach dem Druckversuch Rückschlüsse auf die Dicke der Kapselmembran und die innere Struktur der Kapseln.

### ¹³C- und ¹⁹F-NMR-Spektroskopie

Kernresonanzmessungen an Kohlenstoffatomen unter Beobachtung des ¹³C-Isotops dienen der chemischen Charakterisierung der Kapselwand und liefern Informationen über den Stand des Ablaufs der Polymerisationsreaktion. Darüber hinaus dienen Messungen an den Fluor-Kernen unter Einsatz von gepulsten Feldgradienten der Beobachtung der Selbstdiffusionseigenschaften des fluorierten Kohlenwasserstoffs, woraus wieder auf den Phasenzustand und die Phasenhomogenität des Kapselinhalts geschlossen werden kann. So wird beispielsweise sichergestellt, daß sich der flüssige Inhalt in einer freien Kavität und nicht etwa in einem schwammartig-porösen Medium befindet. Des weiteren ermöglicht die Aufnahme von ¹⁹F-Linienspektren die Beobachtung der Sauerstoffaufnahme: Sauerstoff beeinflußt durch seine paramagnetischen Eigenschaften das Resonanzsignal der Fluor-Kerne, so daß sich typische Linienverbreiterungen und Verkürzungen der Relaxationszeiten ergeben, deren Ausprägung direkte Rückschlüsse auf die Sauerstoffkonzentration im Kapselinneren ermöglicht. Auf diese Weise kann sowohl die Kinetik der Sauerstoffaufnahme als auch die Position des Verteilungsgleichgewichts bestimmt werden.

In der nachfolgenden Tabelle sind die Parameter der hergestellten und charakterisierten Kapseln bzw. der sie enthaltenden Dispersionen wiedergegeben:

| **Parameter** | **Werte** |
|---|---|
| Dichte der Kapseln | 1,80 - 1,95 g/ml |
| Konzentration an Kapseln | 5 - 60 Vol.-% |
| Größe der Kapseln | 50 - 5.000 nm |
| Spezifische Lösungsviskosität η | 0,25 - 1,25 s |
| pH-Wert der Dispersionen | 7,00 - 7,45 |
| Ionenstärke der Dispersionen | 280 - 310 mosmol/l |
| Kapselmaterialien (Material der Kapselhülle) | A: Alkylcyanacrylat |
| | B: Perfluoralkylcyanacrylat |
| | C: Poly(D,L-lactid-co-glycolid) |
| | D: Poly(D,L-lactid-co-glycolid) derivatisiert mit perfluorierten Verbindungen |
| | E: Poly(D,L-lactid-co-glycolid) derivatisiert mit Polyethylenglykol |
| Perfluorcarbone | Perfluordekalin |
| | Perfluoroctan |
| | Perfluordekan |
| Eingesetzte Menge | 5 bis 15.000 ml, insbesondere 5 bis 10.000 ml, vorzugsweise 10 bis 3.000 ml |

Testung der Biokompatibilität der Perfluorcarbon-Nanokapseln, ihrer Aufnahme in Zellen und ihrer Fähigkeit zum Sauerstofftransport/zur Sauerstoffversorgung in einfachen biologischen Systemen

An kultivierten Makrophagen und Endothelzellen, d. h. Zellen des retikuloendothelialen Systems, werden mögliche cytotoxische Effekte der Perfluorcarbon-Nanokapseln, ihre Aufnahme in die Zellen sowie die Aktivierung/Inaktivierung der Zellen durch Kontakt mit den Perfluorcarbon-Nanokapseln mit etablierten Techniken untersucht. Zum Nachweis der cytotoxischen Wirkung der Perfluorcarbon-Nanokapseln sowie ihrer Aufnahme in die Zellen wird unter anderem die Laser-Scanning-Mikroskopie eingesetzt. Die mögliche cytotoxische Wirkung der Perfluorcarbon-Nanokapseln und ihre Aufnahme in Zellen wird auch an *in vivo* perfundierten Rattenlebern studiert. Zum Nachweis der Aufnahme der Nanokapseln (fluoreszenzmarkiert mit Nilrot) in die Zellen des intakten Organs kommt die Intravitalmikroskopie zum Einsatz. An *in vivo* perfundierten Rattenlebern wird auch vergleichend die Fähigkeit von Perfluorcarbon-Nanokapseln zum Sauerstofftransport bzw. zur Sauerstoffversorgung von Geweben untersucht. Aufgrund der geringen physikalischen Löslichkeit von Sauerstoff in wäßrigen Phasen erfordert die übliche Perfusion mit Carbogen (95 % O₂, 5 % CO₂) gesättigtem Krebs-Henseleit-Puffer hohe Perfusionsvolumina (30 ml/min), um eine ausreichende Sauerstoffversorgung zu gewährleisten. In Versuchen mit Perfluorcarbon-Nanokapseln sowie Perfluorcarbon-Emulsionen bzw. Perfluorcarbon-Dispersionen wird bestätigt, daß (im Vergleich zum Krebs-Henseleit-Puffer) die Durchflußrate signifikant gesenkt werden kann, bevor es zu einer ischämischen Gewebeschädigung kommt, bzw. daß eine physiologische Durchflußrate erreichbar ist.

## Patentansprüche

1. Dispersion von künstlichen Sauerstoffträgern, insbesondere als Blutersatzstoff, vorzugsweise zu Zwecken der Transfusion, wobei die Dispersion Kapseln mit reversibler Sauerstoffspeicherfähigkeit enthält, wobei die Kapseln ein sauerstoffpermeables Kapselmaterial umfassen, welches fluorierte, insbesondere perfluorierte Kohlenwasserstoffe, vorzugsweise Perfluorcarbone, enthält und/oder einschließt, wobei das sauerstoffpermeable Kapselmaterial ein sauerstoffpermeables organisches Polymer umfasst oder hieraus besteht, wobei das Polymer ausgewählt ist aus der Gruppe von Poly(lactid-co-glycoliden) und Polyalkylcyanacrylaten sowie deren Mischungen, wobei das organische Polymer frei von Sulfidbrücken ausgebildet ist und eine kohärente Struktur aufweist, wobei in den Kapseln das Gewichtsverhältnis von sauerstoffpermeablem Kapselmaterial zu fluorierten Kohlenwasserstoffen im Bereich von 75 : 25 bis 10 : 90 liegt, wobei die fluorierten Kohlenwasserstoffe Molekulargewichte im Bereich von 250 bis 2.000 g/mol aufweisen und flüssig ausgebildet sind, wobei die Kapseln einen Durchmesser im Bereich von 50 bis 1.000 nm aufweisen, wobei die Dispersion die Kapseln in volumenbezogenen Mengen von 1 bis 70 Vol.-% enthält, bezogen auf die Dispersion, wobei die Dispersion auf einen physiologischen pH-Wert eingestellt ist und eine spezifische Viskosität η im Bereich von 0,1 bis 1,8 s aufweist, bezogen auf Temperaturen im Bereich von 10 °C bis 40 °C, und wobei die Dispersion eine wässrig-basierte Dispersion ist und/oder als kontinuierliche Phase Wasser in Form einer physiologischen und/oder isotonischen Natriumchloridlösung enthält, wobei die Dispersion lagerstabil ist.

2. Dispersion nach Anspruch 1, **dadurch gekennzeichnet,**
**dass** die Kapseln als Kern/Hülle-Kapseln ausgebildet sind, insbesondere wobei das sauerstoffpermeable Kapselmaterial die Kapselhülle ausbildet und/oder insbesondere wobei die fluorierten Kohlenwasserstoffe von der sauerstoffpermeablen Kapselhülle umgeben und/oder eingeschlossen sind und/oder die fluorierten Kohlenwasserstoffe das Innere der Kern/Hülle-Kapseln bilden; oder
**dass** die Kapseln als Matrixkapseln vorliegen, insbesondere wobei das sauerstoffpermeable Kapselmaterial eine Matrix ausbildet und die fluorierten Kohlenwasserstoffe in die Matrix auf Basis des sauerstoffpermeablen Kapselmaterials eingelagert sind, vorzugsweise in homogener und/oder gleichmäßiger Verteilung.

3. Dispersion nach einem der vorangehenden Ansprüche, **dadurch gekennzeichnet, dass** das sauerstoffpermeable Kapselmaterial, insbesondere die Kapselhülle der Kern/Hülle-Kapseln oder das Matrixmaterial der Matrixkapseln, vorzugsweise das insbesondere sauerstoffpermeable organische Polymer der Kapselhülle oder Matrix, eine Modifizierung, insbesondere Oberflächenmodifizierung, und/oder funktionelle Gruppen aufweist, insbesondere wobei die Modifizierung, insbesondere Oberflächenmodifizierung, und/oder die funktionellen Gruppen ausgewählt ist/sind aus (i) aggregationsverhindernden funktionellen Gruppen, (ii) die Wechselwirkung mit biologischen Molekülen steuernden funktionellen Gruppen, (iii) sauren funktionellen Gruppen, (iv) Hydroxylgruppen, (v) unter physiologischem pH-Wert vorzugsweise anionischen Gruppen, insbesondere Carbonsäure-, Sulfat-, Sulfonat-, Phosphat- und Phosphonatgruppen, (vi) Polyalkylenpolyolgruppen, insbesondere Polyethylenglykolgruppen, (vii) Emulgatoren, (viii) Dispergiermitteln sowie deren Kombinationen und/oder Mischungen untereinander.

4. Dispersion nach einem der vorangehenden Ansprüche, **dadurch gekennzeichnet,**
**dass** die Kapseln einen Durchmesser im Bereich von 50 bis 975 nm aufweisen.

5. Dispersion nach einem der vorangehenden Ansprüche, **dadurch gekennzeichnet,**
**dass** in den Kapseln das Gewichtsverhältnis von sauerstoffpermeablem Kapselmaterial zu fluorierten Kohlenwasserstoffen höchstens 50:50 beträgt und/oder dass in den Kapseln das Gewichtsverhältnis von sauerstoffpermeablem Kapselmaterial zu fluorierten Kohlenwasserstoffen im Bereich von 70 : 30 bis 15 zu 85 variiert; und/oder
**dass** die Kapseln eine Dichte von 1,5 bis 2,5 g/ml, insbesondere 1,8 bis 2 g/ml, vorzugsweise 1,85 bis 1,9 g/ml, aufweisen.

6. Dispersion nach einem der vorangehenden Ansprüche, **dadurch gekennzeichnet,**
**dass** die fluorierten Kohlenwasserstoffe zur reversiblen Speicherung und/oder Aufnahme von Sauerstoff, insbesondere unter physiologischen Bedingungen, imstande sind und/oder dass die fluorierten Kohlenwasserstoffe ein reversibles Sauerstoffspeichervermögen, insbesondere unter physiologischen Bedingungen, besitzen; und/oder
**dass** die fluorierten Kohlenwasserstoffe Molekulargewichte im Bereich von 300 bis 1.000 g/mol, bevorzugt 400 bis 600 g/mol, aufweisen; und/oder
**dass** die fluorierten Kohlenwasserstoffe mindestens ein von Fluor verschiedenes Halogen, vorzugsweise Brom, enthalten und/oder dass die fluorierten Kohlenwasserstoffe ausgewählt sind aus der Gruppe von Perfluoroctan, Perfluordekan, Perfluordekalin und Bromperfluoroctan sowie deren Mischungen, insbesondere Perfluoroctan, Perfluordekan und Perfluordekalin sowie deren Mischungen, vorzugsweise Perfluoroctan und/oder Perfluordekan.

7. Dispersion nach einem der vorangehenden Ansprüche, **dadurch gekennzeichnet,**
**dass** die Dispersion die Kapseln in volumenbezogenen Mengen von 5 bis 65 Vol.-%, vorzugsweise 10 bis 60 Vol.-%, besonders bevorzugt 20 bis 50 Vol.-%, enthält, bezogen auf die Dispersion; und/oder
**dass** die Dispersion die Kapseln in massebezogenen Mengen von 5 bis 80 Gew.-%, insbesondere 10 bis 75 Gew.-%, vorzugsweise 15 bis 65 Gew.-%, enthält, bezogen auf die Dispersion; und/oder
**dass** die Dispersion auf eine physiologische Ionenstärke und/oder auf eine physiologische Osmolarität und/oder auf eine physiologische Ionenzusammensezung eingestellt ist; und/oder
**dass** die Dispersion auf einen pH-Wert im Bereich von 6,5 bis 7,9, insbesondere 7 bis 7,5, vorzugsweise 7 bis 7,45, besonders bevorzugt 7,37 bis 7,45, eingestellt ist und/oder dass die Dispersion einen Puffer, insbesondere einen physiologischen Puffer, vorzugsweise auf Phosphatbasis, enthält; und/oder
**dass** die Dispersion eine spezifische Viskosität η im Bereich von 0,2 bis 1,5 s, vorzugsweise 0,25 bis 1,25 s, aufweist, insbesondere bezogen auf Temperaturen im Bereich von 10 °C bis 40 °C.

8. Dispersion nach einem der vorangehenden Ansprüche, **dadurch gekennzeichnet,**
**dass** die Dispersion eine Osmolarität im Bereich von 250 bis 350 mosmol/l, insbesondere 280 bis 310 mosmol/l, aufweist; und/oder
**dass** die Dispersion außerdem übliche Hilfs- und/oder Zusatzstoffe enthält, insbesondere ausgewählt aus der Gruppe von Dispergierhilfsmitteln, Emulgataren, Streckmitteln, Bindemitteln, Netzmitteln, Stabilisierungsmitteln sowie deren Mischungen, vorzugsweise in Konzentrationen von ≤ 50 µmol/l, insbesondere ≤ 40 µmol/l, vorzugsweise ≤ 35 µmol/l.

9. Verwendung einer Dispersion gemäß den vorangehenden Ansprüchen zur Herstellung eines Arzneimittels zur prophylaktischen und/oder therapeutischen Behandlung von Sauerstoffmangelzuständen des menschlichen oder tierischen Körpers.

10. Verwendung einer Dispersion gemäß den vorangehenden Ansprüchen, insbesondere Verwendung nach Anspruch 9, als Blutersatzstoff, insbesondere zu Transfusionszwecken.

11. Verwendung einer Dispersion gemäß den vorangehenden Ansprüchen, insbesondere Verwendung nach Anspruch 9 oder 10, zur Herstellung eines Arzneimittels zur prophylaktischen und/oder therapeutischen Behandlung von Zuständen des menschlichen oder tierischen Körpers bei und/oder nach Blutverlust, insbesondere operativen Eingriffen, Unfällen, Verletzungen oder dergleichen, oder aber zur Herstellung eines Arzeimittels zur prophylaktischen und/oder therapeutischen Behandlung von ischämischen Zuständen oder von Zuständen nach Reperfusion, insbesondere des Tourniquet-Syndroms (Reperfusions-Syndroms), oder zum Schutz von Organen bei der Transplantation bzw. zum Schutz von zu transplantierenden Organen, insbesondere im Zeitraum zwischen Organentnahme und Implantation, insbesondere unter Perfusion.

12. Verwendung einer Dispersion gemäß den vorangehenden Ansprüchen, insbesondere Verwendung nach Anspruch 9 oder 10, zur Herstellung eines Arzneimittels zur prophylaktischen und/oder therapeutischen Behandlung von Gasbläschenbildungen im Blut oder von Gasembolien des menschlichen oder tierischen Körpers.

13. Verwendung einer Dispersion gemäß den vorangehenden Ansprüchen, insbesondere Verwendung nach Anspruch 9 oder 10, zur Anwendung bei Herz/Lungen-Maschinen, insbesondere zur Herstellung eines Arzneimittels zur prophylaktischen und/oder therapeutischen Behandlung von Gasembolien des menschlichen oder tierischen Körpers, oder aber zur Herstellung eines Arzneimittels zur prophylaktischen und/oder therapeutischen Behandlung von Gasvergiftungen oder Rauchvergiftungen des menschlichen oder tierischen Körpers.

14. Verwendung nach einem der vorangehenden Ansprüche, **dadurch gekennzeichnet,**
**dass** die Dispersion in Mengen von 5 bis 15.000 ml, insbesondere 5 bis 10.000 ml, vorzugsweise 10 bis 3.000 ml, je Einzelgabe appliziert wird.

## Claims

1. Dispersion of artificial oxygen carriers in particular as a blood substitute, preferably for transfusion purposes, wherein the dispersion capsules have reversible oxygen storage capability, wherein the capsules comprise an oxygen-permeable capsule material, which contains and/or includes fluorinated, in particular perfluorinated, hydrocarbons, preferably pertluorocarbons, wherein the said oxygen-permeable capsule material comprises or consists of an oxygen-permeable organic polymer, wherein the said polymer is selected from the group consisting of poly(lactide-co-glycolides) and poly alkyl cyanacrylates, and mixtures thereof, wherein the said organic polymer is free of sulfide bridges and has a coherent structure, wherein the weight ratio of oxygen permeable capsule material to fluorinated hydrocarbons in the capsules lies in the range of 75 : 25 to 10 : 90, wherein the fluorinated hydrocarbons have molecular weights in the range from 250 to 2000 g/mol and are liquid, wherein the capsules have a diameter in the range from 50 to 1000 nm, wherein the capsules contain the dispersion in volume-related amounts of from 1 to 70 vol.-% based on the dispersion, wherein the dispersion is adjusted to a physiological pH value and has a specific viscosity η in the range from 0.1 to 1.8 s, based on temperatures in the range from 10 °C to 40 °C, and wherein the dispersion is an aqueous-based dispersion and/or contains water as a continuous phase in the form of a physiological and/or isotonic sodium chloride solution, wherein the dispersion is storage-stable.

2. Dispersion according to claim 1,
**characterized in that**
the capsules are designed as core/shell capsules, in particular wherein the oxygen-permeable capsule material forms the capsule shell and/or in particular wherein the fluorinated hydrocarbons are surrounded and/or enclosed by the oxygen-permeable capsule shell, and/or the fluorinated hydrocarbons form the interior of the core/shell capsules; or
the capsules are present as matrix capsules, in particular wherein the oxygen-permeable capsule material forms a matrix and the fluorinated hydrocarbons are incorporated into the matrix based on the oxygen-permeable capsule material, preferably in homogeneous and/or uniform distribution.

3. Dispersion according to one of the preceding claims,
**characterized in that**
the oxygen-permeable capsule material, in particular the capsule shell of the core/shell capsules or the matrix material of the matrix capsules, preferably the, in particular, oxygen-permeable organic polymer of the capsule shell or matrix, comprises a modification, in particular a surface modification, and/or functional groups, in particular wherein the modification, in particular the surface modification, and/or the functional groups is/are selected from (i) aggregation-preventing functional groups, (ii) functional groups controlling interaction with biological molecules, (iii) acidic functional groups, (iv) hydroyxyl groups, (v) preferably anionic groups under physiological pH value, in particular carboxylic acid, sulfate, sulfonate, phosphate and phosphonate groups, (vi) polyalkylene polyol groups, in particular polyethylene glycol groups, (vii) emulsifiers, (viii) dispersing agents as well as combinations thereof and/or mixtures thereof.

4. Dispersion according to one of the preceding claims,
**characterized in that**
the capsules have a diameter in the range from 50 to 975 nm.

5. Dispersion according to one of the preceding claims,
**characterized in that**
the weight ratio of oxygen-permeable capsule material to fluorinated hydrocarbons in the capsules is at most 50 : 50, and/or that the weight ratio of oxygen-permeable capsule material to fluorinated hydrocarbons in the capsules varies in the range from 70 : 30 to 15 to 85; and/or
the capsules have a density of 1.5 to 2.5 g/ml, in particular 1.8 to 2 g/ml, preferably 1.85 to 1.9 g/ml.

6. Dispersion according to one of the preceding claims,
**characterized in that**
the fluorinated hydrocarbons are capable of reversibly storing and/or absorbing oxygen, in particular under physiological conditions, and/or the fluorinated hydrocarbons have a reversible oxygen storage capability, in particular under physiological conditions; and/or
the fluorinated hydrocarbons have molecular weights in the range from 300 to 1,000 g/mol, preferably from 400 to 600 g/mol; and/or
the fluorinated hydrocarbons contains a halogen different from fluor, preferably bromine, and/or the fluorinated hydrocarbons are selected from the group consisting of perfluorooctane, perfluorodecane, perfluorodecalin and bromo perfluorooctane as well as mixtures thereof, in particular perfluorooctane, perfluorodecane and perfluorodecalin as well as mixtures thereof, preferably perfluorooctane and/or perfluorodecane.

7. Dispersion according to one of the preceding claims,
**characterized in that**
the capsules contain the dispersion in volume-related amounts of 5 to 65 vol.-%, preferably 10 to 60 vol.-%, particularly preferably 20 to 50 vol.-%, based on the dispersion; and/or
the capsules contain the dispersion in mass-based amounts of from 5 to 80 wt.-%, in particular from 10 to 75 wt.-%, preferably from 15 to 65 wt.-%, based on the dispersion; and/or
the dispersion is adjusted to a physiological ionic strength and/or to a physiological osmolarity and/or to a physiological ion composition; and/or
the dispersion is adjusted to a pH value in the range from 6.5 to 7.9, in particular from 7 to 7.5, preferably from 7 to 7.45, particularly preferably from 7.37 to 7.45, and/or the dispersion contains a buffer, in particular a physiological buffer, preferably phosphate-based; and/or
the dispersion has a specific viscosity η in the range from 0.2 to 1.5 s, preferably 0.25 to 1.25 s, in particular based on temperatures in the range from 10 °C to 40 °C.

8. Dispersion according to one of the preceding claims,
**characterized in that**
the dispersion has an osmolarity in the range from 250 to 350 mosmol/l, in particular from 280 to 310 mosmol/l; and/or
the dispersion additionally comprises conventional auxiliaries and/or additives, in particular selected from the group consisting of dispersing auxiliaries, emulsifiers, extenders, binders, wetting agents, stabilizing agents and mixtures thereof, preferably in concentrations of ≤ 50 µmol/l, in particular ≤ 40 µmol/l, preferably ≤ 35 µmol/l.

9. Use of a dispersion according to the preceding claims for the preparation of a medicament for the prophylactic and/or therapeutic treatment of oxygen deficiency states of the human or animal body.

10. Use of a dispersion according to the preceding claims, in particular use according to claim 9, as a blood substitute, in particular for transfusion purposes.

11. Use of a dispersion according to the preceding claims, in particular use according to claim 9 or 10, for the production of a medicament for the prophylactic and/or therapeutic treatment of conditions of the human or animal body during and/or after loss of blood, in particular surgical interventions, accidents, injuries, or the like, or for the preparation of a medicament for the prophylactic and/or therapeutic treatment of ischemic conditions or of conditions after reperfusion, in particular of tourniquet syndrome (reperfusion syndrome), or for the protection of organs during transplanting or protection of organs to be transplanted, in particular in the period between organ collection and implantation, in particular under perfusion.

12. Use of a dispersion according to the preceding claims, in particular use according to claim 9 or 10, for the production of a medicament for the prophylactic and/or therapeutic treatment of gas bubble formation in the blood or of gas embolisms of the human or animal body.

13. Use of a dispersion according to the preceding claims, in particular use according to claim 9 or 10, for use in heart/lung machines, in particular for the production of a medicament for the prophylactic and/or therapeutic treatment of gas embolisms of the human or animal body, or for the production of a medicament for the prophylactic and/or therapeutic treatment of gas poisoning or smoke poisoning of the human or animal body.

14. Use according to one of the preceding claims,
**characterized in that**
the dispersion is applied in amounts of 5 to 15,000 ml, in particular 5 to 10,000 ml, preferably 10 to 3,000 ml, per individual dose.

## Revendications

1. Dispersion de transporteurs d'oxygène artificiels, en particulier en tant que substitut sanguin, de préférence à des fins de transfusion, la dispersion contenant des capsules ayant une capacité réversible de stockage de l'oxygène, les capsules comprenant un matériau de capsule perméable à l'oxygène, qui contient ou comprend des hydrocarbures fluorés, en particulier perfluorés, de préférence des perfluorocarbures, le matériau de capsule perméable à l'oxygène comprenant un polymère organique perméable à l'oxygène ou en étant constitué, le polymère étant choisi dans le groupe des poly(lactide-co-glycolides) et des poly(cyanoacrylates d'alkyle), ainsi que des mélanges de ceux-ci, le polymère organique étant constitué exempt de ponts sulfure et présentant une structure cohérente, le rapport en poids, dans les capsules, entre le matériau de capsule perméable à l'oxygène et les hydrocarbures fluorés étant compris dans la plage de 75:25 à 10:90, les hydrocarbures fluorés présentant une masse moléculaire comprise dans la plage de 250 à 2000 g/mol et ayant une constitution liquide, les capsules présentant un diamètre compris dans la plage de 50 à 1000 nm, la dispersion contenant les capsules en des quantités, rapportées au volume, de 1 à 70 % en volume par rapport à la dispersion, la dispersion étant ajustée à un pH physiologique et présentant une viscosité spécifique η comprise dans la plage de 0,1 à 1,8 s, pour des températures comprises dans la plage de 10°C à 40°C, et la dispersion étant une dispersion à base aqueuse et/ou contenant en tant que phase continue de l'eau sous forme d'une solution physiologique et/ou isotonique de chlorure de sodium, la dispersion étant stable au stockage.

2. Dispersion selon la revendication 1, **caractérisée en ce que**
les capsules sont réalisées sous forme de capsules coeur/gaine, en particulier le matériau de capsule perméable à l'oxygène formant la gaine de la capsule et/ou en particulier les hydrocarbures fluorés étant entourés par la gaine de capsule perméable à l'oxygène et/ou y étant inclus, et/ou les hydrocarbures fluorés formant l'intérieur des capsules coeur/gaine ; ou
les capsules se présentent sous forme de capsules à matrice, et les hydrocarbures fluorés sont incorporés dans la matrice à base du matériau de capsule perméable à l'oxygène, de préférence selon une répartition homogène et/ou uniforme.

3. Dispersion selon l'une des revendications précédentes, **caractérisée en ce que** le matériau de capsule perméable à l'oxygène, en particulier la gaine de capsule des capsules coeur/gaine, ou le matériau de matrice des capsules à matrice, de préférence le polymère organique, en particulier perméable à l'oxygène, de la gaine de la capsule ou de la matrice, présente une modification, en particulier une modification en surface, et/ou des groupes fonctionnels, en particulier la modification, en particulier la modification en surface et/ou les groupes fonctionnels étant choisis parmi (i) les groupes fonctionnels empêchant une agrégation, (ii) les groupes pilotant l'interaction avec les molécules biologiques, (iii) les groupes fonctionnels acides, (iv) les groupes hydroxyle, (v) les groupes anioniques, de préférence à pH physiologique, en particulier les groupes acide carboxylique, sulfate, sulfonate, phosphate et phosphonate, (vi) les groupes polyalkylènepolyol, en particulier les groupes polyéthylèneglycol, (vii) les émulsifiants, (viii) les dispersants, ainsi que les combinaisons et/ou mélanges de ceux-ci les uns avec les autres.

4. Dispersion selon l'une des revendications précédentes, **caractérisée en ce que** les capsules présentent un diamètre compris dans la plage de 50 à 975 nm.

5. Dispersion selon l'une des revendications précédentes, **caractérisée en ce que**
dans les capsules, le rapport en poids entre le matériau de capsule perméable à l'oxygène et les hydrocarbures fluorés est d'au plus 50:50, et/ou que, dans les capsules, le rapport en poids entre le matériau de capsule perméable à l'oxygène et les hydrocarbures fluorés varie dans la plage de 70:30 à 15:85 ; et/ou
les capsules présentent une masse volumique de 1,5 à 2,5 g/ml, en particulier de 1,8 à 2 g/ml, de préférence de 1,85 à 1,9 g/ml.

6. Dispersion selon l'une des revendications précédentes, **caractérisée en ce que**
les hydrocarbures fluorés sont aptes à un stockage et/ou à une absorption réversibles de l'oxygène, en particulier dans les conditions physiologiques, et/ou que les hydrocarbures fluorés possèdent une capacité réversible de stockage de l'oxygène, en particulier dans les conditions physiologiques ; et/ou
que les hydrocarbures fluorés présentent une masse moléculaire comprise dans la plage de 300 à 1000 g/mol, en particulier de 400 à 600 g/mol ; et/ou
que les hydrocarbures fluorés contiennent au moins un halogène différent du fluor, de préférence le brome, et/ou que les hydrocarbures fluorés sont choisis dans le groupe consistant en le perfluoroctane, le perfluorodécane, la perfluorodécaline et/ou le bromoperfluoroctane, ainsi que les mélanges de ceux-ci, en particulier le perfluoroctane, le perfluorodécane et la perfluorodécaline, ainsi que les mélanges de ceux-ci, de préférence le perfluoroctane et/ou le perfluorodécane.

7. Dispersion selon l'une des revendications précédentes, **caractérisée en ce que**
la dispersion contient les capsules en des quantités, rapportées au volume, de 5 à 65 % en volume, de préférence de 10 à 60 % en volume, d'une manière particulièrement préférée de 20 à 50 % en volume, par rapport à la dispersion ; et/ou
que la dispersion contient les capsules en des quantités, rapportées à la masse, de 5 à 80 % en poids, en particulier de 10 à 75 % en poids, de préférence de 15 à 65 % en poids, par rapport à la dispersion ; et/ou
que la dispersion est ajustée à une force ionique physiologique et/ou à une osmolarité physiologique et/ou à une composition ionique physiologique ; et/ou
que la dispersion est ajustée à un pH compris dans la plage de 6,5 à 7,9, en particulier de 7 à 7,5, de préférence de 7 à 7,45, d'une manière particulièrement préférée de 7,37 à 7,45, et/ou que la dispersion contient un tampon, en particulier un tampon physiologique, de préférence à base de phosphate ; et/ou
que la dispersion présente une viscosité spécifique η comprise dans la plage de 0,2 à 1,5 s, de préférence de 0,25 à 1,25 s, en particulier pour des températures comprises dans la plage de 10°C à 40°C.

8. Dispersion selon l'une des revendications précédentes, **caractérisée en ce que**
la dispersion présente une osmolarité comprise dans la plage de 250 à 350 mosmol/l, en particulier de 280 à 310 mosmol/l ; et/ou
que la dispersion contient par ailleurs des adjuvants et/ou additifs usuels, en particulier choisis dans le groupe des auxiliaires de dispersion, des émulsifiants, des diluants, des liants, des mouillants, des stabilisants, ainsi que des mélanges de ceux-ci, de préférence à des concentrations ≤ 50 µmol/l, en particulier ≤ 40 µmol/l, de préférence ≤ 35 µmol/l.

9. Utilisation d'une dispersion selon les revendications précédentes pour la fabrication d'un médicament destiné au traitement prophylactique et/ou thérapeutique d'états de carence en oxygène du corps humain ou animal.

10. Utilisation d'une dispersion selon l'une des revendications précédentes, en particulier utilisation selon la revendication 9, en tant que substitut sanguin, en particulier à des fins de transfusion.

11. Utilisation d'une dispersion selon l'une des revendications précédentes, en particulier utilisation selon la revendication 9 ou 10, pour la fabrication d'un médicament destiné au traitement prophylactique et/ou thérapeutique d'états du corps humain ou animal lors et/ou après une perte sanguine, en particulier après des interventions opératoires, des accidents, des blessures ou analogues, ou cependant pour la fabrication d'un médicament destiné au traitement prophylactique et/ou thérapeutique d'états ischémiques ou d'états après reperfusion, en particulier du syndrome du tourniquet (syndrome de reperfusion), ou pour la protection d'organes lors de la transplantation ou pour la protection d'organes à transplanter, en particulier au cours du laps de temps entre le prélèvement d'un organe et son implantation, en particulier sous perfusion.

12. Utilisation d'une dispersion selon les revendications précédentes, en particulier utilisation selon la revendication 9 ou 10, pour la fabrication d'un médicament destiné au traitement prophylactique et/ou thérapeutique de la formation de bulles de gaz dans le sang ou d'embolies gazeuses du corps humain ou animal.

13. Utilisation d'une dispersion selon les revendications précédentes, en particulier utilisation selon la revendication 9 ou 10, pour une utilisation dans les coeurs-poumons artificiels, en particulier pour la fabrication d'un médicament destiné au traitement prophylactique et/ou thérapeutique d'embolies gazeuses du corps humain ou animal, ou cependant pour la fabrication d'un médicament destiné au traitement prophylactique et/ou thérapeutique d'intoxications par un gaz ou des fumées du corps humain ou animal.

14. Utilisation selon l'une des revendications précédentes, **caractérisée en ce que** la dispersion est administrée en des quantités de 5 à 15 000 ml, en particulier de 5 à 10 000 ml, de préférence de 10 à 3000 ml, par prise individuelle.
